# DEMANDE DE BREVET EUROPEEN

(11) **EP 0 957 169 A1**
(43) Date de publication de la demande: **17.11.1999**
(21) Numéro de dépôt: 98201311.2
(22) Date de dépôt: 22.04.1998
(51) Int. Cl.: C12N 15/52, C12P 19/18, C12N 9/10, C08B 37/00, C12Q 1/68, C12N 1/21

(54) **Identification de gènes de Lactobacillus helveticus LH59 impliqués dans la biosynthèse d'exopolysaccharides**

(71) Demandeur: SOCIETE DES PRODUITS NESTLE S.A., 1800 Vevey (CH)
(72) Inventeur: Stingele, Francesca, 1018 Lausanne (CH)
(74) Mandataire: Van Malderen, Michel

(57) **Abrégé**

Enzyme recombinée, susceptible d'être impliquée dans la biosynthèse de l'EPS ayant l'unité saccharidique répétitive 
β-D-Gal*p*-(1→4)-β-D-Glc*p* 1
↓
3
→5)-β-D-Gal*f*-(1→3)-α-D-Gal*p*-(1→3)-α-D-Glc*p*-(1→3)-β-D-Glc*p*-(1→
 catalysant spécifiquement l'une des liaisons suivantes entre sucres: la liaison osidique α-1,3 entre le carbone 1 d'un D-Glc*p* activé et le carbone 3 d'un Glc*p* appartenant à l'extrémité non-réductrice d'une chaîne saccharidique en formation ; la liaison osidique α-1,3 entre le carbone 1 d'un D-Gal*p* activé et le carbone 3 d'un Glc*p* présent à l'extrémité non-réductrice d'une chaîne saccharidique en formation ;la liaison osidique β-1,3 entre le carbone 1 d'un D-Gal*f* activé et le carbone 3 d'un Gal*p* présent à l'extrémité non-réductrice d'une chaîne saccharidique en formation ; la liaison osidique β-1,3 entre le carbone 1 d'un D-Glc*p* activé et le carbone 3 d'un Gal*f* présent à l'extrémité non-réductrice d'une chaîne saccharidique en formation ; la liaison osidique β-1,4 entre le carbone 1 d'un D-Gal*p* activé et le carbone 4 du Glc*p* présent à l'extrémité non-réductrice d'une chaîne saccharidique en formation ; et, la liaison osidique β-1,5 entre les unités saccharidiques répétitives de l'EPS ci-dessus. L'invention concerne aussi tout ADN recombinant codant pour une enzyme selon l'invention, ainsi que leur utilisation pour la synthèse d'EPS, notamment des EPS ayant sur la chaîne principale de leur unité saccharidique une ou plusieurs ramifications lactose liées à un sucre de la chaîne principale par leur glucose.

LISTE DE SEQUENCES

(1) INFORMATIONS GENERALES:
(i) DEPOSANT:
(A) NOM: SOCIETE DES PRODUITS NESTLE
(B) RUE: 55 avenue nestle
(C) VILLE: vevey
(D) ETAT OU PROVINCE: vaud
(E) PAYS: suisse
(F) CODE POSTAL: 1500
(ii) TITRE DE L' INVENTION: BACTERIES LACTIQUES PRODUISANT DES
EXOPOLYSACCHARIDES
(iii) NOMBRE DE SEQUENCES: 11
(iv) FORME DECHIFFRABLE PAR ORDINATEUR:
(A) TYPE DE SUPPORT: Floppy disk
(B) ORDINATEUR: IBM PC compatible
(C) SYSTEME D' EXPLOITATION: PC-DOS/MS-DOS
(D) LOGICIEL: PatentIn Release #1.0, Version #1.30 (OEB)

(2) INFORMATIONS POUR LA SEQ ID NO: 1:
(i) CARACTERISTIQUES DE LA SEQUENCE:
(A) LONGUEUR: 9482 paires de bases
(B) TYPE: nucl,otide
(C) NOMBRE DE BRINS: double
(D) CONFIGURATION: linéaire
(ii) TYPE DE MOLECULE: ADN (génomique)
(ix) CARACTERISTIQUE:
(A) NOM/CLE: CDS
(B) EMPLACEMENT:1053..1730
(D) AUTRES INFORMATIONS:/product= "ORF eps1"
(ix) CARACTERISTIQUE:
(A) NOM/CLE: CDS
(B) EMPLACEMENT:1734..2849
(D) AUTRES INFORMATIONS:/product= "ORF eps2"
(ix) CARACTERISTIQUE:
(A) NOM/CLE: CDS
(B) EMPLACEMENT:2852..3943
(D) AUTRES INFORMATIONS:/product= "ORF eps3"
(ix) CARACTERISTIQUE:
(A) NOM/CLE: CDS
(B) EMPLACEMENT:3930..5084
(D) AUTRES INFORMATIONS:/product= "ORF eps4"
(ix) CARACTERISTIQUE:
(A) NOM/CLE: CDS
(B) EMPLACEMENT:5077..6096
(D) AUTRES INFORMATIONS:/product= "ORF eps5"
(ix) CARACTERISTIQUE:
(A) NOM/CLE: CDS
(B) EMPLACEMENT:6099..7091
(D) AUTRES INFORMATIONS:/product= "ORF eps6"
(ix) CARACTERISTIQUE:
(A) NOM/CLE: CDS
(B) EMPLACEMENT:7096..8259
(D) AUTRES INFORMATIONS:/product= "ORF eps7"
(ix) CARACTERISTIQUE:
(A) NOM/CLE: CDS
(B) EMPLACEMENT:8284..8910
(D) AUTRES INFORMATIONS:/product= "ORF eps8"

(xi) DESCRIPTION DE LA SEQUENCE: SEQ ID NO: 1:

CTGCAGATTT GTGACATTAG CTGAATATTT GTGCTATATT GACTAAAAAG TGTGTTTATT 60
TTACAGAAAA GTACTATAAA TACGCGTTGT GCTAGTTAAA TCGTTCTAGA CAATAAGCCA 120
AATTATAAGC TAAAATTGCA ATTTCTAACC GGCTTTGAAA GCCTATCAGA CTACGTGCTC 180
GATTGTTCTC GGCATTGTAA TAGGTCAGAA GCGAAAAGTC GCTTTCTATT GTTCTGCGAA 240
TAGCCATCAA TTGATGATCA TTGTGCTTTT TAGCTCCTGT CATATTTTTA CGATATGGTG 300
TCCAAAGTTC ATAACCCATT TGTTTTAGCT GTTGATGCAG TTCTTTGCCT AAATAGCCTT 360
CGTCGCCAAG AAGATAGTAA TTAGATGGAT GGGTATTTTC TATCAGTTCA ACTGTCTCCC 420
TGGCATCATG AACTGATGCT TTTGTTACGA CATAATCAAG AATGTAACCG TCATCGCTAA 480
AATAGCATGA ACTTTGAAAC CATAGAAGTA AATTTCCTTG GTGGCCTTAT AACCAATGTT 540
GGCATAACCG CGAAAAATTT TAGCACGATA GTTGCGAATT GGTTGGCAAA CAGGTACCGG 600
AAAGCTGTCA ATGATCAAGA ACTGTCCATT CAGGTTCAAC CTTTTTATTC ATTTCTTGCC 660
GTATCTGATA AATCAATTGC AATAGCTGAC GTGAACGTCG ATTAAAACGT GAGTGTGATA 720
AACAATTGAA ACATTCACAG AATCTTCTTT GTGATTCAAT TCCTGTCTTA GCTTGCCAGA 780
TAAGTAAAGC CAAAATCAGA CTGTCCGTAG TTTTAATTTG ATCAATATTT CGCCGATGAG 840
TAAACTCAGC CGGTGCATAC AAACGATACC AGTGCCGACA AATTATCACT AAATCTTTAA 900
AACTAACTTG TAAATGGTGG CTAAAACGCT TAAGCTTAAG GCAGTTCAAT CAGATCAGAC 960
TCTTTTCTTT TTGATACTAC AACTTGAGTC TAGTCCGATT GGACTTTTTT ATTAACTAAA 1020
ACGATTTAAC TAGCACCACG CGTTATAAAT ATATGACATT GAGGGTAAGA GGAAAAATGA 1080
TGGTAATGGA GAAACTAAAA GTTAATCCGG CTAAAGTACA TGGGCGGTAT GTTTATCACG 1140
CAGTGAAAAG ATTGTTTGAT ATTATTGCTA GTGTAGTCGG ATTGATTCTA CTGTCTCCAT 1200
TATTTTTATT TCTAATAATA AAAATAAGAC ATGAAGACGG TGGACCAGCA TTCTATTCAC 1260
AAGAGAGAAT TGGTAAAGAT GAAAAACTAT TTAAGATGTG GAAGTTTCGT TCAATGGTTG 1320
TAAACGCTGA TCAAATGATT GATCAATTAG AGGATCAAAA CGAAATTGAA GGTGCAATGT 1380
TCAAAATTAA GGATGATCCA AGAATTACTA AGATTGGGCA TACTATTCGT AAGTACAGTC 1440
TTGATGAGTT GCCACAGCTC TGGAATGTGT TAATCGGTGA CATGTCTCTT GTGGGACCAC 1500
GCCCGCCTCT ACCATCAGAA GTAGAAGAGT ATACCGATTA TGACAAGCAG CGACTGCTTG 1560
TCATGCCAGG TTGCACAGGG TTATGGCAAG TTACCAAGCG TAACGAAGCT GACTTTGATG 1620
AAATGGTTTG GCTTGATATC ATGTATATCA ACCATTCTGG CTTGTGGGAG GACTTTAAGT 1680
TGATTATTAA AACAGTATTA GTTATGATTC ATCCGAATGG AGCCTATTAA AGTATGAAAA 1740
ATAAAATACG AGTGCTTCAT GTGGCAGAGG CTGCAGGTGG AGTAGAAAGA TATTTAGAAA 1800
CACTATTTAA ATATAGTAGA GATAAGGTTG AAAATATCTT AGTATGTTCA CAAAATTATG 1860
ACTATAAGAA GTTTAAAAAG CTTACGAGCA GAGTAATTGT CTTAAAAATG GCTCATAATA 1920
TTCAACCATC TTCAGATATC CAAGTTGAAA AAACTCTGAG AAAGATTATT AAGCAATTAA 1980
AGCCTGATAT AGTCTATGCA CATTCAAGTA AAGCTGGCGC TTTTGCTAGG ATTGCTGATT 2040
TGGGCTTAAA AAATAAGATC ATCTATAATC CTCACGGCTG GGCTTTTAAT ATGCAACAAT 2100
CTGCTATGAA AAAGAAAATG TATAAATGGG TTGAAAAAAT TTCAGCCCAT TTTTGCGATA 2160
AGATAGTTTG TATCTCTGAT GCTGAAAGAG AATCAGCTCT TAGAGAAAAA ATCTGTAAGC 2220
CAAGTAAATT ACAGGTGATC TATAACGGAA TTGACTTAGA AGAGATAGAG AAGACTACTC 2280
CTATAAGTAG AGCTCAATTG GGTATCCCTG AAGATGCTTT TGTTGTAGGT ATGGTTGGGA 2340
GACTATCTAA ACAAAAGGCT CCAGATACCT TTGTAAAGTC TGCAAAGTTG ATTAAAGAGA 2400
AAATCCCTAA CGCATTTTTC TTGATGGTTG GAGATGGCGA GTTACGAGAT CAGATTGAAA 2460
GCTTAATTAA TCAATATGAT CTTGGCTCAT CATTCTTAAT AACTGGCTGG GTAGACAATC 2520
CTACAGCCTA TATGAAGATT ATGGATGTGG GGATGCTACT ATCTCGTTGG GAAGGCTTTG 2580
GTTTAGTTCT ACCAGAGTAT ATGGCTTGTG GAGTGCCTAT TGTAGCTACT AATGTGGATG 2640
CTATTCCTAA TATCGTTACG AATGGTGTTA ATGGCATGCT AGTTGATAAA GATGATTACC 2700
ATAAAGCAGC AGATGCAGTC GATAAACTAT TTAAACAACC TGATTTGAGA TCTTCTTTAG 2760
TTAAAACTGA AAGTAATATT GTAAAAAATA AGTTTGATGG TAAAAGAGTC GCACAAAATA 2820
GTGAACAATT ATATAAAGAG TTGATATAAA GATGATTCGA GTACTTCATA GTGAATTACA 2880
AAGCAATATT GGAGGCATTG AGGCTTTCTT ACTAAACTTG ACGAAAACAA TAGATAAAAC 2940
TAATTTACAA TTTGATTTTT TAATGCGTGG TAACAACTCA TATTTGGAAA CGACGCTGAA 3000
GGATTATGGA GCCAATATTT ATAAAGTACC CTCTGGAATA ATCAATTACT ATAAGTTCGT 3060
AAAAAATATT CTCATTAAGA ATGATTATGA TTTTGTTCAT GTTCATAAAA ATTCGGCAGC 3120
TAATATAGTG TTGCCATTGA TGGTAAAAAA GTATACTAAT ACAAAATTAA TTGTTCATTC 3180
TCATAATACT AGTCCTAGTA GTGGTTCTAA GATTGCAATT ATGTTGCATA AAATTAATAG 3240
AAATAAATTA ATGCAATTAA GTGATTATCG TTTTGCATGT TCAGATACTG CTGCGGAGTG 3300
GATGTTTGGT CATGATTATA AGGATGAACA AGTAAAAATA ATAAAGAATG GTATTATTAC 3360
TAAAGACTAT GTTTATGATC CTCAAATGAG AAAACGGATG CGTCGGCAAT TAGGCTTAAA 3420
AGGTAAGTTT GTCATAGGAC ATGTTGGGGC TTTCAGAGAG CAAAAAAATC ACAAATTTTT 3480
ACTTAATGTT TTCTCAAAAT CAAATATTCC TAATGCAGAG CTGGTGTTAG TTGGTGATGG 3540
AATTTTAAAA GAAAAAATTG TGGCTCAAGC ACAAGAATTA GGAATCAAAA ATAAAATTAA 3600

GTTTTTAGGT AGTAGAAATG ATATTAATGA AATCTTACAA AGTTTTGATT TATTTGTAAT 3660
GCCTTCTTTA TGGGAAGGAT TATCTGTTTC TGCTATAGAA GCACAAGCTA GTGGACTTCA 3720
ATTGATTTTA TCTGAAAATG TATCACATTC AATCAAAATC ACGCCAAATG TAAAATTTCT 3780
AAATTTAAAT GATATTGATA GTTGGATAAG AGAAATAAAA TCAAAAGTTG TTGATGATAA 3840
ATTTGATCGA AAAGATGAAA CAAAAGCTAT CAGTAAATAT GGTTATGATA TGAGTAATAG 3900
TGCTCAGATA ATTAGAAGGA TTTATTCAAA TGAAGAAATA TAGAATATTG GTGTGGGGAT 3960
TTACTGAAAA TATGGGCGGA GTAGAAAATG TGATTTTAAA TTATTATCAA AATTTTGATA 4020
ATAGAATAGC CATAGATTTT ATGGCTGAAT CTAAGAAAAA AATGGCCTAT GAAGATTTAT 4080
TACGTCGCTC AGGTAGTAGG GTATTTCATT TGCCCAGAAA GAGATATCAT TTATTAGAAT 4140
ATTATCAAGC ATTAAAAAGA TTCTTCTCAG AACATGGTTC TGATTATGAT TGTGTTTGGA 4200
GTAATGTCAA TAACCTATCA AATATAGATA TATTGGTATA CGCAAAAAGA TTTGGCGTTA 4260
AAAGACGAAT AATCCATAGT CATAATTCAA AGAATATGTT TAAAGGTATA GCACGATATC 4320
AAAGAGGTAT TATGCAATGG AAAAATAAAT TTATAATTTC CAAATATGCT ACAGATTACT 4380
GGGCCTGCTC AAAAGACGCT GCGGATTATG TATTTCCTGC AAAGGTTCTT TCTCAAGTAA 4440
AAATAATAAG AAATGCAATT GAAGTATCAA AATATCGATT TGATGAACAA AAAGAAAAAA 4500
AGATAAGAAA GCAGTATAAT TTAGAAGGTC ATTTTGTTAT AGGAAATGTA GGAAGATTGC 4560
ATTTTCAAAA AAATCAAATA TATATATTGA AAATTTTAAA GAAAATAAAA AATGATATTC 4620
CTACTGCCAA ATTAGTTTTT GTTGGAGATG GACCAGATAA AAATAACTTA TATGATGTAG 4680
CTGAAAAATT AAACGTCAAA GATGATGTGA TTTTTGCCGG ATCTCAGGCA AAAATAGAAG 4740
ACTGGTATAG TGCTTTTGAT ATTTTTTTAT TTCCTTCATT GTTTGAAGGC TTTGGCAATG 4800
CATTGATTGA AGCGCAGGCT AATGGATTGA TGATATTAGC ATCTCAAAAT GTAATTCCAA 4860
AAGAAACAAT AATAAACTCC AATTATAAAA TGATTCCTTT AGATAAAGGT AGTAACTATT 4920
GGGCTAAAAT AATTGTTGAT AATTTTAAAG TGTTTAATCA CAAAATACCT AGGGAATTAG 4980
ATGAAAATAT TAAGTATAAT TTTGAAAATA AAGGATATAA CAGTGAAACT AATGCTGTTA 5040
AAGTTGAAGA TTTATTATTA AAGGACAAAA GTAAACATGG ATAATCCATT AATTTCAGTT 5100
ATTGTTCCTG CATATAATGC AGAAAAAAGT ATCACTAAAT GCATTTTTAG TTTATTGCGA 5160
CAAAACATTA AAGAATCATA CAAAATAATA ATTGTTAATG ATGGATCAAC AGATGATACT 5220
TTAACAGAAA TAAAAAGTTT TAAAGACAAT CCTAAAATTT CAATTATTGA TAAAAAAAAT 5280
GAAGGTGTTT CTATAGCACG AAATACAGCA TTAAAAAATG TTAATAGTAA ATTTGTTACA 5340
TTTGTAGACT CAGATGATTT TGTAGATAGT AATTTTTTGG TAGATTTGTA TTCTGGCTTT 5400
AAAGATGCAA AAGATGTTGA TCTAACAATA TCAGGATTTA GAAAATTGAA TACTAATAAT 5460
AAATTACTGG AAGAATATAA GTTTTCAAAC GTCACTCGTT CTGCTGAGGA TGTAATTTCT 5520
TTGCTACTTA ATGAGACTGG ACCACAAGGA TATCTATGGA ATAAGATGTT TCGAACATCA 5580
ATTATAAAAA AATATAGTTT ACTATTAGAT CCTAAAATTT TTATGGCAGA AGATTTATTA 5640
TTTTGTATAC AGTATCTTAA ATTTTCTAAA AAAGTGCGTG TTAGTAACTA TTGTGATTAC 5700
AATTATGTTC AAGACAGTAA TAGTATGAAT GGTGGACTAA GTTTTACTAA AAATAATAGA 5760
CGATATAAAA AAATCTTTAA TAATTATTTT TTTGCTTTAA ATAAAATATT GAATGAAATT 5820
AAGAATAATC CAAAAAAATA TAGTTTAGCG ATTATTAATG TAAAGGCAAG ATTGGCTAGA 5880
GAGTATAGTA TTTTTTTAAG ATTGTTATTA CTTAATAATG ATAAAAATAA GAAATTACAA 5940
AAAGAAGTAT ACTGGCAGGC CTATAAATTA AAAAGATATG TTTTTAAAAG TAAGAGTATA 6000
CCAGTGTCTA AGAAGATGTT TTTTGCATGT ACTATATATT GCCCTCACTT GGTGCATATA 6060
AGTGATGAAG CGAGATTTAG TTGGGAAAGG AATTAATTGT GAAACCAAAA GTTACTATCA 6120
TAGTACCTAT TTATAACGTT GAAAAATATA TAGAAAAATG CCTTCGAAGT TTACTGGCTC 6180
AATCATTCTC TGAATTTGAA GTATGGGCTA TAGATGATGG GTCTCCTGAT AACTCAAGTG 6240
ATATTGTAAA AGAATTTTTT AAAAAAGATT CTAGAATAAA ATTAATTCAA AAGGAAAATG 6300
GCGGTTATGG ATCGGTTCTG GAATATGCAA TTAAAAGAAT AGAAACAGAA TATTTTTTAA 6360
TTTGTGATCC CGATGATTGG TTAGAAGAAA ATGCTGTCGA AACGTTATAT AAAAATGCAA 6420
GTGATAATAA ATTAGACATT GTAGTTGCTG ATAAATATGA TCATTATAGT TTAACTAATG 6480
AAAAAATGTA TAGTTCGTTC GCTCCGTCTA ATTACAAGAT AGAAGCTGAT AAAGTTTATA 6540
CAACATCATT AGAAATCCAA AAATTTGCAT TTGGTTACGT ATCACCTCAC GCTAAATTAT 6600
ATAAAACTAT AATAGCGAAA AATATTGAAT TCCCAAGAAA AGTCAGTTTT ACAGATTATT 6660
TATTGTTCAT AGTAGCCTTA TCTAAGCAAA AATCTATAAT GTATGTAAAT CAACCATTAG 6720
CAGATTACTT AATAGATAGA CCTGGAAATA CAACAACTGC AAAAAATAAA AAGAAAATAG 6780
AAGATCTTTG TACGGTATGG GACAGTACTT TTAATCAAAT TAAAGATACC AATCAGAGCA 6840
TTTTAATGTA CAGAATGTTT ACGCAATTAA AATTTATACT TTTAGAGATT TATAAATTAA 6900
ATCCTGTATT AATTAGTACA TATAATTTAA ATGTATACAC GAGAGCAAAC ATACTAAAAA 6960
AATATTATAA AGAAATTCTA AAAGTAATAC CTAATTTTGA ACAAAAAATA ATTTTTAGAG 7020
GCATTGAATC TAAGTATTTA CAGAAGATAT TTGTTAAATT ATTTTGTAAA TTAAAGCAAA 7080
AGGATAGTTA AATTTATGTC AAATAGTACC TATATACAAA TTTATAAGTT ACTTTTATAT 7140
TTTTTATTGA CATTAGCTAT GATAACTAAT GGGCAAAGTG CTCTATATAT TATATTAGTC 7200
GTAACTATCA TAATGTTGAT TCTGTATATT AATCAGCCTA AAAAGAAAAT GTACAGTAAA 7260
ACTATATATA CTAATATAGT ATTTCAAATT GTGTTAGTTT TAGTAGAATA TTTTAGATTT 7320

AAAAATACGA TATTATTTTA TCAGTGGGAC AATTCTATAT TTCGTTTTTA TGGAGTTATC 7380
TTCTGTTTAT TGTTAGTTCC ACCATTGTAT GAAGTCATTT GTGAAGATAA AGATATTTTT 7440
TTAAAAAATT TAAATCGATT AGGTCTTATA GTATTATTTG TTCGAGTAAT GAGTTGGTAT 7500
ATCTATAATA CCAGAGGTTT GGATATTGGT TTTACTTTTT TACAGGGTAG ACCTGAATGG 7560
GTAAAATCGA TGGCAGGTAT TCCTTTAATA CGTATGCCAG GTATGTTTTT AGAAGGATTT 7620
TTATTATGCT ATAGTGTTGC ATTTCTTTTG AAATCAAAGG GGAAGATTAA AGTAGCATAT 7680
TTTATAGAAA TACTGTTTTT ATTCTTTTAT GAATGGGTTG TTTTTCAATC ACGTGGGTAT 7740
TTGATTATTT ATACTTTAGT GATATATATC ACATTTATTT ACTATTTAAT ACATAACCGA 7800
AAAATAATAG CATGTATTAG TATACTTGTT TTAGGAATAC TTTTGCTTAG TTTATTTCAT 7860
AATATGATAT TTTCATCGTT TTCTACTACT GGGACTTACG CAGTTTCCAC AAATACTAGA 7920
ATATTAGAAT ATAGTTACTT TCCCGAGTTA TGGAACCGAA GTAGTATTAT ATGGGGATTT 7980
GGACTAACTC CTGATACATT GGAAGGAATT GTTACGATTA GTGATATGAA TTTCATTATA 8040
AATTTGTATC AATTTGGAAT TATTGGGTTA TTAATTAGAA TAAGTCCATT TGTAGTAGGA 8100
TTTATTATCT TACTTAAATA TAAGAAAGAA AAAATATTCT TTTTAGCATT CAATCTTTTT 8160
TGTTTATTCT TTTCAATATT TTTCAATCCA TATATGTACA TGACTATTTT TTTACTTCCA 8220
TTATATTTAT CATACAGTAT GTATGTAGAA TATAAATAGA TTAGTTTTTA TTAGAGGATA 8280
TTAATGAGAT ACACATATAT ATCATTTTTA GGATCGGACG ATTATTTAAT AGGAATACTA 8340
GCTTTAAAAA ATAGGTTAGA TGTATTAAAA AGCGAGTATC CAGTAACTGT ATTAGTTTCA 8400
GAAGATATAA GTAATAAATC TAAAAGAATA TTAAAGAAAT ATGATATTCC TTATGTAATA 8460
AAGAAAGATT ATGTACCGTC TGAAGAAATT CAAAAAAATA ATACATCTTT TGGGATGCCA 8520
CAGTGGTCTA AAACTTTTGG TAAGTTGTAT ATTTTTGGCA TGACTGAATT TGATAAGCTA 8580
GTTTATGTAG ATGCAGATAT GTTAATTAAA AAGAATATTG ATGAATTATT TCAAAATAAG 8640
AATCTATCCG CTGTTATTGC TGGAGTAAAA TATCCAGGTA ATGAAAACTG GTCACTAACC 8700
TTAAATTCGG GATTAATGGT TATTGTTCCT AAAAAGAATG AGGATGTACG TTTATTTGAA 8760
TTAATTGAAT CAAATAAATT TGAAAAAGGT GGAGATCAAG ACATTATTCA TCTAGCATAT 8820
CCTAATTCGA GTGAGAATGA ACGACTGCAT TTAAAAGAGG AATATAATTT ATTAGCACCT 8880
TATGAATTGT ATTATGTATC AAAAAATTAG TAAATTCTGA TAAGTTAAAA GTAATACATT 8940
ATATTGGGGC GACAAAACCG TGGATGCGTT CACAATTGTC AGAATATAAA CACATAATAG 9000
GAATAATCGT GAGCGGATTA TGTATTAGCA GTTCTTTAAA GGGAATAAAA TTTGCCGTGC 9060
AAGATTATAT TTTTTATTGC AAGTTGTGCA GGAAAATAGA AAATGATATC TCCAAAATGG 9120
AATAGTTAAG AGGAAAAATG AATTATTTAG TAGTGGGTTC TGGTCTCTAT GGGTCAACGT 9180
TTGCTCACGA GGCTGCAAAT TATGGAAACA AAGTTACAGT AATTGAAAAA AGAGATCATA 9240
TTGCAGGTAA TATCTACACT AAAGAAATGG ATGGTATCCA AGTTCATATG TATGGTGCTC 9300
ACATTTTCCA TACTTCTAAT AAGAAGGTTT GGGATTATGT TCATCAATTT GCGGACTTTA 9360
ACCGCTATAC TAATAGCCCT GTTGCCAATT ACAAAGGGCA TATGTATAAC TTGCCATTCA 9420
ATATGAATAC CTTTAGTGAA ATGTGGGGTG TTAGAACACC ACAAGAAGCT ATGGCTAAGA 9480
TC 9482

(2) INFORMATIONS POUR LA SEQ ID NO: 2:
(i) CARACTERISTIQUES DE LA SEQUENCE:
(A) LONGUEUR: 225 acides amin,s
(B) TYPE: acide aminé
(C) NOMBRE DE BRINS: simple
(D) CONFIGURATION: linéaire
(ii) TYPE DE MOLECULE: protéine
(vi) ORIGINE:
(A) ORGANISME: EPS1
(xi) DESCRIPTION DE LA SEQUENCE: SEQ ID NO: 2:

Met Thr Leu Arg Val Arg Gly Lys Met Met Val Met Glu Lys Leu Lys
1               5                   10                  15
Val Asn Pro Ala Lys Val His Gly Arg Tyr Val Tyr His Ala Val Lys
            20                  25                  30
Arg Leu Phe Asp Ile Ile Ala Ser Val Val Gly Leu Ile Leu Leu Ser
        35                  40                  45
Pro Leu Phe Leu Phe Leu Ile Ile Lys Ile Arg His Glu Asp Gly Gly
    50                  55                  60
Pro Ala Phe Tyr Ser Gln Glu Arg Ile Gly Lys Asp Glu Lys Leu Phe
65                  70                  75                  80

Lys Met Trp Lys Phe Arg Ser Met Val Val Asn Ala Asp Gln Met Ile
                85                  90                  95
Asp Gln Leu Glu Asp Gln Asn Glu Ile Glu Gly Ala Met Phe Lys Ile
            100                 105                 110
Lys Asp Asp Pro Arg Ile Thr Lys Ile Gly His Thr Ile Arg Lys Tyr
        115                 120                 125
Ser Leu Asp Glu Leu Pro Gln Leu Trp Asn Val Leu Ile Gly Asp Met
    130                 135                 140
Ser Leu Val Gly Pro Arg Pro Pro Leu Pro Ser Glu Val Glu Glu Tyr
145                 150                 155                 160
Thr Asp Tyr Asp Lys Gln Arg Leu Leu Val Met Pro Gly Cys Thr Gly
                165                 170                 175
Leu Trp Gln Val Thr Lys Arg Asn Glu Ala Asp Phe Asp Glu Met Val
            180                 185                 190
Trp Leu Asp Ile Met Tyr Ile Asn His Ser Gly Leu Trp Glu Asp Phe
        195                 200                 205
Lys Leu Ile Ile Lys Thr Val Leu Val Met Ile His Pro Asn Gly Ala
    210                 215                 220
Tyr
225

(2) INFORMATIONS POUR LA SEQ ID NO: 3:
    (i) CARACTERISTIQUES DE LA SEQUENCE:
        (A) LONGUEUR: 371 acides aminés
        (B) TYPE: acide aminé
        (C) NOMBRE DE BRINS: simple
        (D) CONFIGURATION: linéaire
   (ii) TYPE DE MOLECULE: protéine
   (vi) ORIGINE:
        (A) ORGANISME: EPS2
   (xi) DESCRIPTION DE LA SEQUENCE: SEQ ID NO: 3:

Met Lys Asn Lys Ile Arg Val Leu His Val Ala Glu Ala Ala Gly Gly
1               5                   10                  15
Val Glu Arg Tyr Leu Glu Thr Leu Phe Lys Tyr Ser Arg Asp Lys Val
            20                  25                  30
Glu Asn Ile Leu Val Cys Ser Gln Asn Tyr Asp Tyr Lys Lys Phe Lys
        35                  40                  45
Lys Leu Thr Ser Arg Val Ile Val Leu Lys Met Ala His Asn Ile Gln
    50                  55                  60
Pro Ser Ser Asp Ile Gln Val Glu Lys Thr Leu Arg Lys Ile Ile Lys
65                  70                  75                  80
Gln Leu Lys Pro Asp Ile Val Tyr Ala His Ser Ser Lys Ala Gly Ala
                85                  90                  95
Phe Ala Arg Ile Ala Asp Leu Gly Leu Lys Asn Lys Ile Ile Tyr Asn
            100                 105                 110
Pro His Gly Trp Ala Phe Asn Met Gln Gln Ser Ala Met Lys Lys Lys
        115                 120                 125
Met Tyr Lys Trp Val Glu Lys Ile Ser Ala His Phe Cys Asp Lys Ile
    130                 135                 140
Val Cys Ile Ser Asp Ala Glu Arg Glu Ser Ala Leu Arg Glu Lys Ile
145                 150                 155                 160
Cys Lys Pro Ser Lys Leu Gln Val Ile Tyr Asn Gly Ile Asp Leu Glu
                165                 170                 175
Glu Ile Glu Lys Thr Thr Pro Ile Ser Arg Ala Gln Leu Gly Ile Pro
            180                 185                 190
Glu Asp Ala Phe Val Val Gly Met Val Gly Arg Leu Ser Lys Gln Lys
        195                 200                 205

Ala Pro Asp Thr Phe Val Lys Ser Ala Lys Leu Ile Lys Glu Lys Ile
    210                 215                 220
Pro Asn Ala Phe Phe Leu Met Val Gly Asp Gly Glu Leu Arg Asp Gln
225                 230                 235                 240
Ile Glu Ser Leu Ile Asn Gln Tyr Asp Leu Gly Ser Ser Phe Leu Ile
                245                 250                 255
Thr Gly Trp Val Asp Asn Pro Thr Ala Tyr Met Lys Ile Met Asp Val
            260                 265                 270
Gly Met Leu Leu Ser Arg Trp Glu Gly Phe Gly Leu Val Leu Pro Glu
        275                 280                 285
Tyr Met Ala Cys Gly Val Pro Ile Val Ala Thr Asn Val Asp Ala Ile
    290                 295                 300
Pro Asn Ile Val Thr Asn Gly Val Asn Gly Met Leu Val Asp Lys Asp
305                 310                 315                 320
Asp Tyr His Lys Ala Ala Asp Ala Val Asp Lys Leu Phe Lys Gln Pro
                325                 330                 335
Asp Leu Arg Ser Ser Leu Val Lys Thr Glu Ser Asn Ile Val Lys Asn
            340                 345                 350
Lys Phe Asp Gly Lys Arg Val Ala Gln Asn Ser Glu Gln Leu Tyr Lys
        355                 360                 365
Glu Leu Ile
    370

(2) INFORMATIONS POUR LA SEQ ID NO: 4:
    (i) CARACTERISTIQUES DE LA SEQUENCE:
        (A) LONGUEUR: 363 acides aminés
        (B) TYPE: acide aminé
        (C) NOMBRE DE BRINS: simple
        (D) CONFIGURATION: linéaire
   (ii) TYPE DE MOLECULE: protéine
   (vi) ORIGINE:
        (A) ORGANISME: EPS3
   (xi) DESCRIPTION DE LA SEQUENCE: SEQ ID NO: 4:

Met Ile Arg Val Leu His Ser Glu Leu Gln Ser Asn Ile Gly Gly Ile
1               5                   10                  15
Glu Ala Phe Leu Leu Asn Leu Thr Lys Thr Ile Asp Lys Thr Asn Leu
            20                  25                  30
Gln Phe Asp Phe Leu Met Arg Gly Asn Asn Ser Tyr Leu Glu Thr Thr
        35                  40                  45
Leu Lys Asp Tyr Gly Ala Asn Ile Tyr Lys Val Pro Ser Gly Ile Ile
    50                  55                  60
Asn Tyr Tyr Lys Phe Val Lys Asn Ile Leu Ile Lys Asn Asp Tyr Asp
65                  70                  75                  80
Phe Val His Val His Lys Asn Ser Ala Ala Asn Ile Val Leu Pro Leu
                85                  90                  95
Met Val Lys Lys Tyr Thr Asn Thr Lys Leu Ile Val His Ser His Asn
            100                 105                 110
Thr Ser Pro Ser Ser Gly Ser Lys Ile Ala Ile Met Leu His Lys Ile
        115                 120                 125
Asn Arg Asn Lys Leu Met Gln Leu Ser Asp Tyr Arg Phe Ala Cys Ser
    130                 135                 140
Asp Thr Ala Ala Glu Trp Met Phe Gly His Asp Tyr Lys Asp Glu Gln
145                 150                 155                 160
Val Lys Ile Ile Lys Asn Gly Ile Ile Thr Lys Asp Tyr Val Tyr Asp
                165                 170                 175
Pro Gln Met Arg Lys Arg Met Arg Arg Gln Leu Gly Leu Lys Gly Lys
            180                 185                 190

Phe Val Ile Gly His Val Gly Ala Phe Arg Glu Gln Lys Asn His Lys
        195                 200                 205
Phe Leu Leu Asn Val Phe Ser Lys Ser Asn Ile Pro Asn Ala Glu Leu
    210                 215                 220
Val Leu Val Gly Asp Gly Ile Leu Lys Glu Lys Ile Val Ala Gln Ala
225                 230                 235                 240
Gln Glu Leu Gly Ile Lys Asn Lys Ile Lys Phe Leu Gly Ser Arg Asn
                245                 250                 255
Asp Ile Asn Glu Ile Leu Gln Ser Phe Asp Leu Phe Val Met Pro Ser
            260                 265                 270
Leu Trp Glu Gly Leu Ser Val Ser Ala Ile Glu Ala Gln Ala Ser Gly
        275                 280                 285
Leu Gln Leu Ile Leu Ser Glu Asn Val Ser His Ser Ile Lys Ile Thr
    290                 295                 300
Pro Asn Val Lys Phe Leu Asn Leu Asn Asp Ile Asp Ser Trp Ile Arg
305                 310                 315                 320
Glu Ile Lys Ser Lys Val Val Asp Asp Lys Phe Asp Arg Lys Asp Glu
                325                 330                 335
Thr Lys Ala Ile Ser Lys Tyr Gly Tyr Asp Met Ser Asn Ser Ala Gln
            340                 345                 350
Ile Ile Arg Arg Ile Tyr Ser Asn Glu Glu Ile
        355                 360

(2) INFORMATIONS POUR LA SEQ ID NO: 5:
    (i) CARACTERISTIQUES DE LA SEQUENCE:
        (A) LONGUEUR: 384 acides aminés
        (B) TYPE: acide aminé
        (C) NOMBRE DE BRINS: simple
        (D) CONFIGURATION: linéaire
   (ii) TYPE DE MOLECULE: protéine
   (vi) ORIGINE:
        (A) ORGANISME: EPS4
   (xi) DESCRIPTION DE LA SEQUENCE: SEQ ID NO: 5:

Met Lys Lys Tyr Arg Ile Leu Val Trp Gly Phe Thr Glu Asn Met Gly
1               5                   10                  15
Gly Val Glu Asn Val Ile Leu Asn Tyr Tyr Gln Asn Phe Asp Asn Arg
            20                  25                  30
Ile Ala Ile Asp Phe Met Ala Glu Ser Lys Lys Lys Met Ala Tyr Glu
        35                  40                  45
Asp Leu Leu Arg Arg Ser Gly Ser Arg Val Phe His Leu Pro Arg Lys
    50                  55                  60
Arg Tyr His Leu Leu Glu Tyr Tyr Gln Ala Leu Lys Arg Phe Phe Ser
65                  70                  75                  80
Glu His Gly Ser Asp Tyr Asp Cys Val Trp Ser Asn Val Asn Asn Leu
                85                  90                  95
Ser Asn Ile Asp Ile Leu Val Tyr Ala Lys Arg Phe Gly Val Lys Arg
            100                 105                 110
Arg Ile Ile His Ser His Asn Ser Lys Asn Met Phe Lys Gly Ile Ala
        115                 120                 125
Arg Tyr Gln Arg Gly Ile Met Gln Trp Lys Asn Lys Phe Ile Ile Ser
    130                 135                 140
Lys Tyr Ala Thr Asp Tyr Trp Ala Cys Ser Lys Asp Ala Ala Asp Tyr
145                 150                 155                 160
Val Phe Pro Ala Lys Val Leu Ser Gln Val Lys Ile Ile Arg Asn Ala
                165                 170                 175
Ile Glu Val Ser Lys Tyr Arg Phe Asp Glu Gln Lys Arg Lys Lys Ile
            180                 185                 190

Arg Lys Gln Tyr Asn Leu Glu Gly His Phe Val Ile Gly Asn Val Gly
        195                 200                 205
Arg Leu His Phe Gln Lys Asn Gln Ile Tyr Ile Leu Lys Ile Leu Lys
    210                 215                 220
Lys Ile Lys Asn Asp Ile Pro Thr Ala Lys Leu Val Phe Val Gly Asp
225                 230                 235                 240
Gly Pro Asp Lys Asn Asn Leu Tyr Asp Val Ala Glu Lys Leu Asn Val
                245                 250                 255
Lys Asp Asp Val Ile Phe Ala Gly Ser Gln Ala Lys Ile Glu Asp Trp
            260                 265                 270
Tyr Ser Ala Phe Asp Ile Phe Leu Phe Pro Ser Leu Phe Glu Gly Phe
        275                 280                 285
Gly Asn Ala Leu Ile Glu Ala Gln Ala Asn Gly Leu Met Ile Leu Ala
    290                 295                 300
Ser Gln Asn Val Ile Pro Lys Glu Thr Ile Ile Asn Ser Asn Tyr Lys
305                 310                 315                 320
Met Ile Pro Leu Asp Lys Gly Ser Asn Tyr Trp Ala Lys Ile Ile Val
                325                 330                 335
Asp Asn Phe Lys Val Phe Asn His Lys Ile Pro Arg Glu Leu Asp Glu
            340                 345                 350
Asn Ile Lys Tyr Asn Phe Glu Asn Lys Gly Tyr Asn Ser Glu Thr Asn
        355                 360                 365
Ala Val Lys Val Glu Asp Leu Leu Leu Lys Asp Lys Ser Lys His Gly
    370                 375                 380

(2) INFORMATIONS POUR LA SEQ ID NO: 6:
    (i) CARACTERISTIQUES DE LA SEQUENCE:
        (A) LONGUEUR: 339 acides aminés
        (B) TYPE: acide aminé
        (C) NOMBRE DE BRINS: simple
        (D) CONFIGURATION: linéaire
   (ii) TYPE DE MOLECULE: protéine
   (vi) ORIGINE:
        (A) ORGANISME: EPS5
   (xi) DESCRIPTION DE LA SEQUENCE: SEQ ID NO: 6:

Met Asp Asn Pro Leu Ile Ser Val Ile Val Pro Ala Tyr Asn Ala Glu
1               5                   10                  15
Lys Ser Ile Thr Lys Cys Ile Phe Ser Leu Leu Arg Gln Asn Ile Lys
            20                  25                  30
Glu Ser Tyr Lys Ile Ile Ile Val Asn Asp Gly Ser Thr Asp Asp Thr
        35                  40                  45
Leu Thr Glu Ile Lys Ser Phe Lys Asp Asn Pro Lys Ile Ser Ile Ile
    50                  55                  60
Asp Lys Lys Asn Glu Gly Val Ser Ile Ala Arg Asn Thr Ala Leu Lys
65                  70                  75                  80
Asn Val Asn Ser Lys Phe Val Thr Phe Val Asp Ser Asp Asp Phe Val
                85                  90                  95
Asp Ser Asn Phe Leu Val Asp Leu Tyr Ser Gly Phe Lys Asp Ala Lys
            100                 105                 110
Asp Val Asp Leu Thr Ile Ser Gly Phe Arg Lys Leu Asn Thr Asn Asn
        115                 120                 125
Lys Leu Leu Glu Glu Tyr Lys Phe Ser Asn Val Thr Arg Ser Ala Glu
    130                 135                 140
Asp Val Ile Ser Leu Leu Leu Asn Glu Thr Gly Pro Gln Gly Tyr Leu
145                 150                 155                 160
Trp Asn Lys Met Phe Arg Thr Ser Ile Ile Lys Lys Tyr Ser Leu Leu
                165                 170                 175

Leu Asp Pro Lys Ile Phe Met Ala Glu Asp Leu Leu Phe Cys Ile Gln
            180                 185                 190
Tyr Leu Lys Phe Ser Lys Lys Val Arg Val Ser Asn Tyr Cys Asp Tyr
        195                 200                 205
Asn Tyr Val Gln Asp Ser Asn Ser Met Asn Gly Gly Leu Ser Phe Thr
    210                 215                 220
Lys Asn Asn Arg Arg Tyr Lys Lys Ile Phe Asn Asn Tyr Phe Phe Ala
225                 230                 235                 240
Leu Asn Lys Ile Leu Asn Glu Ile Lys Asn Asn Pro Lys Lys Tyr Ser
                245                 250                 255
Leu Ala Ile Ile Asn Val Lys Ala Arg Leu Ala Arg Glu Tyr Ser Ile
            260                 265                 270
Phe Leu Arg Leu Leu Leu Leu Asn Asn Asp Lys Asn Lys Lys Leu Gln
        275                 280                 285
Lys Glu Val Tyr Trp Gln Ala Tyr Lys Leu Lys Arg Tyr Val Phe Lys
    290                 295                 300
Ser Lys Ser Ile Pro Val Ser Lys Lys Met Phe Phe Ala Cys Thr Ile
305                 310                 315                 320
Tyr Cys Pro His Leu Val His Ile Ser Asp Glu Ala Arg Phe Ser Trp
                325                 330                 335
Glu Arg Asn

(2) INFORMATIONS POUR LA SEQ ID NO: 7:
    (i) CARACTERISTIQUES DE LA SEQUENCE:
        (A) LONGUEUR: 330 acides aminés
        (B) TYPE: acide aminé
        (C) NOMBRE DE BRINS: simple
        (D) CONFIGURATION: linéaire
   (ii) TYPE DE MOLECULE:protéine
   (vi) ORIGINE
        (A) ORGANISME: EPS6
   (xi) DESCRIPTION DE LA SEQUENCE: SEQ ID NO: 7:

Met Lys Pro Lys Val Thr Ile Ile Val Pro Ile Tyr Asn Val Glu Lys
1               5                   10                  15
Tyr Ile Glu Lys Cys Leu Arg Ser Leu Leu Ala Gln Ser Phe Ser Glu
            20                  25                  30
Phe Glu Val Trp Ala Ile Asp Asp Gly Ser Pro Asp Asn Ser Ser Asp
        35                  40                  45
Ile Val Lys Glu Phe Phe Lys Lys Asp Ser Arg Ile Lys Leu Ile Gln
    50                  55                  60
Lys Glu Asn Gly Gly Tyr Gly Ser Val Leu Glu Tyr Ala Ile Lys Arg
65                  70                  75                  80
Ile Glu Thr Glu Tyr Phe Leu Ile Cys Asp Pro Asp Asp Trp Leu Glu
                85                  90                  95
Glu Asn Ala Val Glu Thr Leu Tyr Lys Asn Ala Ser Asp Asn Lys Leu
            100                 105                 110
Asp Ile Val Val Ala Asp Lys Tyr Asp His Tyr Ser Leu Thr Asn Glu
        115                 120                 125
Lys Met Tyr Ser Ser Phe Ala Pro Ser Asn Tyr Lys Ile Glu Ala Asp
    130                 135                 140
Lys Val Tyr Thr Thr Ser Leu Glu Ile Gln Lys Phe Ala Phe Gly Tyr
145                 150                 155                 160
Val Ser Pro His Ala Lys Leu Tyr Lys Thr Ile Ile Ala Lys Asn Ile
                165                 170                 175
Glu Phe Pro Arg Lys Val Ser Phe Thr Asp Tyr Leu Leu Phe Ile Val
            180                 185                 190
Ala Leu Ser Lys Ala Lys Ser Ile Met Tyr Val Asn Gln Pro Leu Ala
        195                 200                 205

Asp Tyr Leu Ile Asp Arg Pro Gly Asn Thr Thr Thr Ala Lys Asn Lys
    210                 215                 220
Lys Lys Ile Glu Asp Leu Cys Thr Val Trp Asp Ser Thr Phe Asn Gln
225                 230                 235                 240
Ile Lys Asp Thr Asn Gln Ser Ile Leu Met Tyr Arg Met Phe Thr Gln
                245                 250                 255
Leu Lys Phe Ile Leu Leu Glu Ile Tyr Lys Leu Asn Pro Val Leu Ile
            260                 265                 270
Ser Thr Tyr Asn Leu Asn Val Tyr Thr Arg Ala Asn Ile Leu Lys Lys
        275                 280                 285
Tyr Tyr Lys Glu Ile Leu Lys Val Ile Pro Asn Phe Glu Gln Lys Ile
    290                 295                 300
Ile Phe Arg Gly Ile Glu Ser Lys Tyr Leu Gln Lys Ile Phe Val Lys
305                 310                 315                 320
Leu Phe Cys Lys Leu Lys Gln Lys Asp Ser
                325                 330

(2) INFORMATIONS POUR LA SEQ ID NO: 8:
    (i) CARACTERISTIQUES DE LA SEQUENCE:
        (A) LONGUEUR: 387 acides aminés
        (B) TYPE: acide aminé
        (C) NOMBRE DE BRINS: simple
        (D) CONFIGURATION: linéaire
   (ii) TYPE DE MOLECULE: protéine
   (vi) ORIGINE:
        (A) ORGANISME: EPS7
   (xi) DESCRIPTION DE LA SEQUENCE: SEQ ID NO: 8:

Met Ser Asn Ser Thr Tyr Ile Gln Ile Tyr Lys Leu Leu Leu Tyr Phe
1               5                   10                  15
Leu Leu Thr Leu Ala Met Ile Thr Asn Gly Gln Ser Ala Leu Tyr Ile
            20                  25                  30
Ile Leu Val Val Thr Ile Ile Met Leu Ile Leu Tyr Ile Asn Gln Pro
        35                  40                  45
Lys Lys Lys Met Tyr Ser Lys Thr Ile Tyr Thr Asn Ile Val Phe Gln
    50                  55                  60
Ile Val Leu Val Leu Val Glu Tyr Phe Arg Phe Lys Asn Thr Ile Leu
65                  70                  75                  80
Phe Tyr Gln Trp Asp Asn Ser Ile Phe Arg Phe Tyr Gly Val Ile Phe
                85                  90                  95
Cys Leu Leu Leu Val Pro Pro Leu Tyr Glu Val Ile Cys Glu Asp Lys
            100                 105                 110
Asp Ile Phe Leu Lys Asn Leu Asn Arg Leu Gly Leu Ile Val Leu Phe
        115                 120                 125
Val Arg Val Met Ser Trp Tyr Ile Tyr Asn Thr Arg Gly Leu Asp Ile
    130                 135                 140
Gly Phe Thr Phe Leu Gln Gly Arg Pro Glu Trp Val Lys Ser Met Ala
145                 150                 155                 160
Gly Ile Pro Leu Ile Arg Met Pro Gly Met Phe Leu Glu Gly Phe Leu
                165                 170                 175
Leu Cys Tyr Ser Val Ala Phe Leu Leu Lys Ser Lys Gly Lys Ile Lys
            180                 185                 190
Val Ala Tyr Phe Ile Glu Ile Leu Phe Leu Phe Phe Tyr Glu Trp Val
        195                 200                 205
Val Phe Gln Ser Arg Gly Tyr Leu Ile Ile Tyr Thr Leu Val Ile Tyr
    210                 215                 220

Ile Thr Phe Ile Tyr Tyr Leu Ile His Asn Arg Lys Ile Ile Ala Cys
225                 230                 235                 240

Ile Ser Ile Leu Val Leu Gly Ile Leu Leu Leu Ser Leu Phe His Asn
                245                 250                 255
Met Ile Phe Ser Ser Phe Ser Thr Thr Gly Thr Tyr Ala Val Ser Thr
            260                 265                 270
Asn Thr Arg Ile Leu Glu Tyr Ser Tyr Phe Pro Glu Leu Trp Asn Arg
        275                 280                 285
Ser Ser Ile Ile Trp Gly Phe Gly Leu Thr Pro Asp Thr Leu Glu Gly
    290                 295                 300
Ile Val Thr Ile Ser Asp Met Asn Phe Ile Ile Asn Leu Tyr Gln Phe
305                 310                 315                 320
Gly Ile Ile Gly Leu Leu Ile Arg Ile Ser Pro Phe Val Val Gly Phe
                325                 330                 335
Ile Ile Leu Leu Lys Tyr Lys Lys Glu Lys Ile Phe Phe Leu Ala Phe
            340                 345                 350
Asn Leu Phe Cys Leu Phe Phe Ser Ile Phe Phe Asn Pro Tyr Met Tyr
        355                 360                 365
Met Thr Ile Phe Leu Leu Pro Leu Tyr Leu Ser Tyr Ser Met Tyr Val
    370                 375                 380
Glu Tyr Lys
385

(2) INFORMATIONS POUR LA SEQ ID NO: 9:
    (i) CARACTERISTIQUES DE LA SEQUENCE:
        (A) LONGUEUR: 208 acides aminés
        (B) TYPE: acide aminé
        (C) NOMBRE DE BRINS: simple
        (D) CONFIGURATION: linéaire
    (ii) TYPE DE MOLECULE: protéine
    (vi) ORIGINE:
        (A) ORGANISME: EPS8
    (xi) DESCRIPTION DE LA SEQUENCE: SEQ ID NO: 9:

Met Arg Tyr Thr Tyr Ile Ser Phe Leu Gly Ser Asp Asp Tyr Leu Ile
1               5                   10                  15
Gly Ile Leu Ala Leu Lys Asn Arg Leu Asp Val Leu Lys Ser Glu Tyr
            20                  25                  30
Pro Val Thr Val Leu Val Ser Glu Asp Ile Ser Asn Lys Ser Lys Arg
        35                  40                  45
Ile Leu Lys Lys Tyr Asp Ile Pro Tyr Val Ile Lys Lys Asp Tyr Val
    50                  55                  60
Pro Ser Glu Glu Ile Gln Lys Asn Asn Thr Ser Phe Gly Met Pro Gln
65                  70                  75                  80
Trp Ser Lys Thr Phe Gly Lys Leu Tyr Ile Phe Gly Met Thr Glu Phe
                85                  90                  95
Asp Lys Leu Val Tyr Val Asp Ala Asp Met Leu Ile Lys Lys Asn Ile
            100                 105                 110
Asp Glu Leu Phe Gln Asn Lys Asn Leu Ser Ala Val Ile Ala Gly Val
        115                 120                 125
Lys Tyr Pro Gly Asn Glu Asn Trp Ser Leu Thr Leu Asn Ser Gly Leu
    130                 135                 140
Met Val Ile Val Pro Lys Lys Asn Glu Asp Val Arg Leu Phe Glu Leu
145                 150                 155                 160
Ile Glu Ser Asn Lys Phe Glu Lys Gly Gly Asp Gln Asp Ile Ile His
                165                 170                 175
Leu Ala Tyr Pro Asn Ser Ser Glu Asn Glu Arg Leu His Leu Lys Glu
            180                 185                 190

Glu Tyr Asn Leu Leu Ala Pro Tyr Glu Leu Tyr Tyr Val Ser Lys Asn
        195                 200                 205

(2) INFORMATIONS POUR LA SEQ ID NO: 10:
(i) CARACTERISTIQUES DE LA SEQUENCE:
(A) LONGUEUR: 22 paires de bases
(B) TYPE: nucléotide
(C) NOMBRE DE BRINS: simple
(D) CONFIGURATION: linéaire
(ii) TYPE DE MOLECULE: Autre acide nucléique
(A) DESCRIPTION: /desc = "amorce nucléotidique"
(xi) DESCRIPTION DE LA SEQUENCE: SEQ ID NO: 10:

GAYCCNMGRT NACNAARATH GG 22

(2) INFORMATIONS POUR LA SEQ ID NO: 11:
(i) CARACTERISTIQUES DE LA SEQUENCE:
(A) LONGUEUR: 23 paires de bases
(B) TYPE: nucléotide
(C) NOMBRE DE BRINS: simple
(D) CONFIGURATION: linéaire
(ii) TYPE DE MOLECULE: Autre acide nucléique
(A) DESCRIPTION: /desc = "amorce nucléotidique"
(xi) DESCRIPTION DE LA SEQUENCE: SEQ ID NO: 11:

GGNCKNGGNC CNACNARNSW CAT 23

## Description

La présente invention se rapporte à de nouvelles fonctions enzymatiques de bactéries lactiques alimentaires, ainsi que de nouveaux enzymes et gènes, impliqués dans la biosynthèse d'exopolysaccharides.

### État de la technique

Il est connu que les bactéries lactiques sont susceptibles de produire dans leur milieu de culture deux classes de polysaccharides, à savoir les homopolysaccharides comme les dextranes ou les levanes qui sont constitués par l'assemblage répété d'un seul sucre, et les hétéropolysaccharides appelés communément exopolysaccharides ou EPS (EPS est l'abréviation du terme "exopolysaccharide") constitués par l'assemblage de plusieurs sucres différents formant une unité répétitive (Cerning J., Bactéries lactiques, Vol I, de Rossart H et Luquet F. M., Lorica, 309-329, 1994).

Une bactérie lactique alimentaire produisant un EPS peut conférer un caractère filant et/ou une texture lisse et crémeuse à un lait acidifié (Cerning *et al*., FEMS Microbiol., 87, 113-130, 19/90). Les EPS peuvent aussi présenter des activités biologiques particulièrement intéressantes pour la santé humaine ou animale, comme des activités anti-tumeurs ou probiotiques, par exemple (Oda M. *et al.*, Agric. Biol. Chem., 47, 1623-1625, 1983; EP94870139.6)

Par ailleurs, l'industrie alimentaire est confrontée à une instabilité génétique de la biosynthèse des EPS dans les bactéries lactiques. Ceci se traduit généralement au cours d'une fermentation par la perte de la production d'EPS par tout ou partie des bactéries lactiques (voir "Cerning J." ci-dessus). Les produits fermentés industriels sont ainsi sujets à des variations dans leur contenu en EPS, ce qui n'est pas toujours acceptable. Pour remédier à ces problèmes, l'industrie recours actuellement à l'isolement et la caractérisation périodique de ses bactéries de manière à séparer celles qui ont perdu leur caractère originel.

On connaît des gènes de bactéries lactiques alimentaires impliqués dans la biosynthèse d'EPS.

WO92/02142 révèle ainsi l'existence du plasmide pHV67 qui produit dans *Lactococcus lactis* subsp. *lactis* (mésophile) une substance capable d'augmenter la viscosité d'un lait fermenté. La structure saccharidique de cette substance n'étant pas connue, les différentes fonctions des enzymes codées par pHV67 sont également inconnues.

US5066588 décrit deux plasmides provenant d'une souche de *Streptococcus cremoris* (mésophile) capable de conférer un caractère épaississant à un *Streptococcus lactis.* La structure saccharidique de cet épaississant n'étant pas connue, les différentes fonctions des enzymes codées par ces plasmides sont également inconnues.

Vescovo *et al.* ont mis en évidence un plasmide d'une souche *Lactobacillus casei* subsp. *casei* (mésophile) codant pour un phénotype Muc+, c'est à dire pour des fonctions liées à la production d'épaississants exocellulaires (Vescovo *et al.,* Biotechnology Letters, Vol II, 709-712, 1989). La structure saccharidique de cet épaississant n'étant pas connue, les différentes fonctions des enzymes codées par ce plasmide sont également inconnues.

EP750043 (Société des Produits Nestlé) décrit un opéron de gènes de la souche *Streptococcus thermophilus* CNCM I-1590 impliqué dans la synthèse d'un EPS ayant la structure répétitive suivante.

→3)-β-D-Glcp(1→3)-α-D-GalpNAc(1→3)-β-D-Galp(1→
6
↑
1
α -D-Galp

Des travaux menés sur ces enzymes (résultats non-publiés) ont permis de montrer que la biosynthèse de cet EPS se fait de manière progressive avec, à chaque étape, l'addition d'une nouvelle unité de sucre qui vient s'attacher par sa fonction semi-acétalique à un hydroxyle alcoolique d'une autre unité de sucre, laquelle est à l'extrémité non-réductrice d'une chaîne de sucres liée à une amorce. Ces enzymes catalysent ou contrôlent ainsi spécifiquement les fonctions suivantes.
(1) La liaison sous forme d'un isomère α ou β entre le carbone 1 (qui porte la fonction réductrice aldéhyde) d'un D-Gal*p* activé et un phosphate d'une amorce lipophilique notamment du genre undécaprénol, ou protéique notamment du genre ACP (Acyl Carrier Protein), par exemple.
(2) La liaison osidique α-1,3 entre le carbone 1 d'un Gal*p*NAc activé (UDP-Gal*p*NAc) et le carbone 3 du D-Gal*p* lié à son amorce.
(3) La liaison osidique β-1,3 entre le carbone 1 d'un D-Glc*p* activé (UDP-D-Glc*p*) et le carbone 3 du sucre présent à l'extrémité non-réductrice de la chaîne β-D-Gal*p*(1→3)-α/β-D-Gal*p*-amorce.
(4) La liaison osidique α-1,6 entre le carbone 1 d'un D-Gal*p* activé et le carbone 6 du sucre présent à l'extrémité non-réductrice de la chaîne β-D-Glc*p*(1→3)β-D-Gal*p*(1→3)-α/β-D-Gal*p*-amorce.
(5) Le transport des unités saccharidiques répétitives de l'EPS décrit ci-dessus à l'extérieur de la cellule bactérienne.
(6) La liaison osidique β-1,3 entre les unités saccharidiques répétitives de l'EPS ci-dessus.
(7) La régulation du nombre d'unités saccharidiques dans l'EPS, et donc de son poids moléculaire final.

Van Kranenburg *et al.* ont aussi décrit un opéron de gènes, présent sur un plasmide d'une souche *Lactococcus lactis,* impliqué dans la synthèse d'un EPS ayant la structure répétitive suivante (Mol. Microbiology, 24, 387-397, 1997).

→4)-β-D-Galp(1→4)-β-D-Glcp(1→4)-β-D-Glcp(1→
2      3
↑      ↑
1      PO4
α-L-Rhap      ↑
1
α-D-Galp

Bien que les enzymes codant pour cet EPS soient maintenant connues, il reste encore à élucider la spécificité de chaque enzyme. Pour le moment on peut valablement supposer, au regard des homologies avec d'autres opérons impliqués dans la biosynthèse de polysaccharides, que la biosynthèse débute par la liaison d'un Glc*p* activé sur une amorce lipophilique ou protéique.

Les connaissances relatives à la biosynthèse des EPS dans les bactéries lactiques alimentaires sont encore néanmoins très fragmentaires. Cependant il est maintenant admis que, basée sur les structures d'EPS élucidées jusqu'à présent et par analogie avec la production de composés tels que les lipopolysaccharides (Whitfield *et al.*, Adv. Microbiol. Physiol., 35, 136-246, 1993), la synthèse débute par l'activation de monomères de sucres (glucose, galactose...) en sucres nucléotidiques utilisés comme précurseurs par les glycosyltransférases. La première étape de la biosynthèse de l'unité répétitive est catalysée par une glycosyltransférase particulière qui reconnaît, d'une part, un précurseur de type sucre nucléotidique et, d'autre part, une amorce lipophilique, notamment du genre undécaprénol-phosphate, ou protéique notamment du genre ACP, par exemple. Les autres glycosyltransférases agissent ensuite séquentiellement en ajoutant un sucre spécifique sur l'unité répétitive en construction. Les unités répétitives complètes sont ensuite exportées vers le milieu extracellulaire avant d'être polymérisées.

A ce jour, il existe toujours un besoin de disposer de nouvelles enzymes impliquées dans la synthèse d'EPS, et notamment des enzymes catalysant des liaisons spécifiques entre des sucres. La présente invention vise à remplir ces besoins.

### Résumé de l'invention

A cet effet, la présente invention concerne toute enzyme recombinée, que l'on peut purifier et qui est susceptible d'être impliquée dans la biosynthèse de l'EPS ayant l'unité saccharidique répétitive 
β-D-Gal*p*-(1→4)-β-D-Glc*p* 1
↓
3
→5)-β-D-Gal*f*-(1→3)-α-D-Gal*p*-(1→3)-α-D-Glc*p*-(1→3)-β-D-Glc*p*-(1→
 catalysant spécifiquement l'une des liaisons suivantes entre sucres:
- la liaison osidique α-1,3 entre le carbone 1 d'un D-Glc*p* activé et le carbone 3 d'un Glc*p* appartenant à l'extrémité non-réductrice d'une chaîne saccharidique en formation, notamment la chaîne suivante: α/β-D-Glc*p*-amorce;
- la liaison osidique α-1,3 entre le carbone 1 d'un D-Gal*p* activé et le carbone 3 d'un Glc*p* présent à l'extrémité non-réductrice d'une chaîne saccharidique en formation, notamment la chaîne suivante: α-D-Glc*p*-(1→3)-β/α-D-Glc*p*-amorce;
- la liaison osidique β-1,3 entre le carbone 1 d'un D-Gal*f* activé et le carbone 3 d'un Gal*p* présent à l'extrémité non-réductrice d'une chaîne saccharidique en formation, notamment la chaîne suivante: α-D-Gal*p*-(1→3)-α-D-Glc*p*-(1→3)-β/α-D-Glc*p*-amorce ;
- la liaison osidique β-1,3 entre le carbone 1 d'un D-Glc*p* activé et le carbone 3 d'un Gal*f* présent à l'extrémité non-réductrice d'une chaîne saccharidique en formation, notamment la chaîne suivante: β-D-Gal*f*-(1→3)-α-D-Gal*p*-(1→3)-α-D-Glc*p*-(1→3)-β-D-Glc*p*-amorce ;
- la liaison osidique β-1,4 entre le carbone 1 d'un D-Gal*p* activé et le carbone 4 du Glc*p* présent à l'extrémité non-réductrice d'une chaîne saccharidique en formation, notamment la chaîne suivante: β-D-Glc*p*-(1→3)-β-D-Gal*f*-(1→3)-α-D-Gal*p*-(1→3)-α-D-Glc*p*-(1→3)-β-D-Glc*p*-amorce ; et,
- la liaison osidique β-1,5 entre les unités saccharidiques répétitives de l'EPS ci-dessus.

Un autre objet de l'invention concerne tout ADN recombiné codant pour une enzyme selon l'invention, et notamment tout vecteur d'ADN ou toute cellule renfermant un tel ADN recombiné, et exprimant une enzyme recombinée fonctionnelle.

Un autre objet de l'invention concerne un procédé de production d'un EPS, dans lequel (1) on clone dans un vecteur un fragment d'ADN codant pour au moins l'une des enzymes selon l'invention, ledit vecteur comprenant en outre une séquence permettant la réplication autonome ou l'intégration dans une cellule hôte, (2) on transforme une cellule hôte par ledit vecteur, ladite cellule hôte utilisant les enzymes codées par le vecteur, le cas échéant en combinaison avec d'autres enzymes produites par la cellule hôte, pour la biosynthèse d'un EPS, (3) puis on cultive la cellule hôte transformée dans des conditions appropriées pour la production d'un EPS.

Un autre objet de l'invention concerne un procédé de synthèse d'un EPS qui est constitué par l'assemblage répété d'une seule unité saccharidique, elle-même formée d'une chaîne principale de sucres dont les extrémités sont impliquées dans la polymérisation des unités, dans lequel on utilise plusieurs enzymes recombinées ou plusieurs gènes recombinants selon l'invention qui permettent la création sur la chaîne principale de l'unité saccharidique d'au moins une ramification lactose liée à un sucre de la chaîne principale par son glucose.

Un dernier objet de l'invention concerne un nouveau polysaccharide branché, constitué par l'assemblage répété d'une seule unité saccharidique, elle-même formée d'une chaîne principale de sucres dont les extrémités sont impliquées dans la polymérisation des unités, caractérisé en ce que la chaîne principale comprend au moins une ramification lactose liée à un sucre de la chaîne principale par son glucose, le galactose de cette ramification étant en outre lié à un N-acétyl-galactosamine, pour autant que ce polysaccharide ne consiste pas en la répétition de l'unité saccharidique suivante

β-D-Gal*p*Nac-(1→4)-β-D-Gal*p*-(1→4)-β-D-Glc*p*
1
↗
3
→5)-β-D-Gal*f*-(1→3)-α-D-Gal*p*-(1→3)-α-D-Glc*p*-(1→3)-β-D-Glc*p*(1→

### Description de la figure

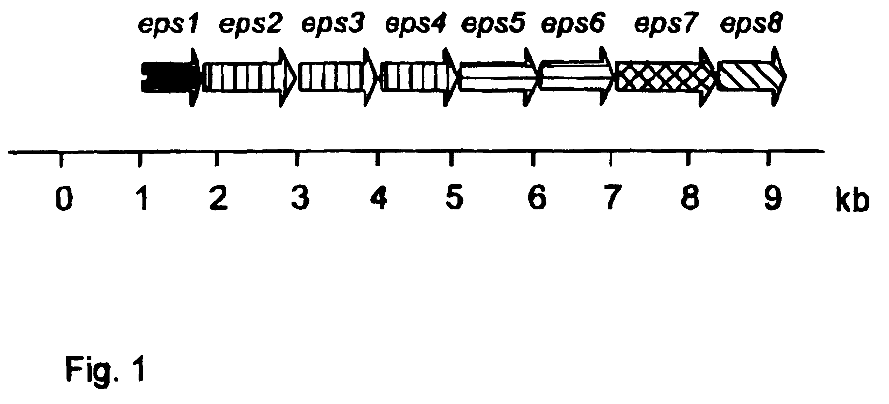

La figure 1 représente la carte physique de l'opéron impliqué dans la synthèse de l'EPS de la souche *S. thermophilus* CNCM 1-1449. Les flèches horizontales indiquent la présence de cadres de lectures (ORF) potentiels. Les noms des gènes correspondants aux ORFs sont indiqués en dessous des flèches.

### Description détaillée de l'invention

Dans la suite de la description, les séquences en nucléotides des gènes ou amorces utilisés, ainsi que les séquences en acides aminés des enzymes recombinées, sont représentées dans la liste de séquences fournie ci-après, et sont nommées, pour des raisons de simplification, sous les sigles numérotés "SEQ ID NO:".

Le terme "EPS" désigne un polymère formé par la condensation d'un grand nombre de molécules de sucres (= oses) de types différents. Cet EPS est plus particulièrement constitué par l'assemblage répété d'une seule unité saccharidique, elle-même formée d'une chaîne principale de sucres dont les extrémités sont impliquées dans la polymérisation des unités. Cette chaîne principale peut comprendre en outre des ramifications de sucres qui ne sont pas impliquées dans la polymérisation des unités, lesdites ramifications étant greffées par des liaisons osidiques sur un ou plusieurs sucres de cette chaîne.

On représente une unité saccharidique en indiquant (1) les sigles usuels des sucres (Glc: glucose; Gal: galactose; Rha: rhamnose; GalNac: N-acétyl-galactosamine; NeuAc: acide N-acétylneuraminique; etc.) ; (2) le type de chaque liaison osidique sous la forme d'une flèche entre les numéros de carbone de deux sucres adjacents (les numéros de carbone attribués sur le cycle d'un sucre sont ceux reconnus par la nomenclature internationale des sucres: IUPAC) ; (3) l'anomérie de chaque liaison osidique sous la forme du sigle α ou β, ledit sigle étant positionné à gauche d'un sucre pour indiquer l'anomérie de la liaison osidique présente à la droite de ce sucre; (4) la nature pyranose ou furanose de chaque sucre en adjoignant respectivement la lettre *p* ou *f* juste après le sigle de chaque sucre ; (5) et la série D ou L de chaque sucre, en positionnant la lettre à gauche du sucre concerné.

Dans une chaîne saccharidique en formation, la réaction directe entre une fonction semi-acétalique d'une nouvelle unité de sucre et un hydroxyle alcoolique du sucre présent à l'extrémité non-réductrice de la chaîne ne se fait pas spontanément, et c'est pourquoi le groupement semi-acétalique doit au préalable être activé, c'est-à-dire rendu plus réactif en vue de la formation de la liaison osidique. Cette activation est catalysée par des enzymes bactériennes formant un sucre nucléotidique, et plus particulièrement un uridine-diphosphate de D-Glc*p* (UDP-Glc*p)*, D-Gal*p* (UDP-Gal*p*) ou D-Gal*f* (UDP-Gal*f*) dans le contexte de la présente invention. Pour initier la formation d'un chaîne saccharidique, un premier sucre activé doit d'abord être lié sur le phosphate d'une amorce lipophilique ou protéique. L'addition d'une nouvelle unité de sucre activé sur la chaîne se fera ensuite par sa fonction semi-acétalique au niveau d'un hydroxyle alcoolique du sucre présent à l'extrémité non-réductrice de cette chaîne, c'est-à-dire à l'extrémité opposée de celle où l'amorce est fixée.

Dans la suite de la description, on représente une chaîne saccharidique en formation, en indiquant les sigles usuels des sucres, les types de liaisons osidiques, la position de l'amorce sur la chaîne, la nature du cycle et de la série D ou L de chaque sucre. Enfin, du fait que l'on ne sait pas encore quel isomère est formé lorsque le premier sucre d'une chaîne est lié à une amorce, par commodité d'écriture, on utilise ainsi le sigle "α/β" afin d'indiquer que l'un des deux isomères est formé.

Au sens de la présente invention, on entend par "séquence homologue" toute séquence nucléotidique ou d'acides aminés, conduisant ou donnant un variant d'enzyme qui catalyse l'assemblage de sucres, ne différant des séquences selon l'invention que par la substitution, la délétion ou l'addition d'un petit nombre de bases nucléotidiques ou d'acides aminés, par exemple 1 à 150 paires de bases (pb) ou 1 à 50 acides aminés. Ces variants d'enzyme peuvent conserver la même spécificité d'assemblage de sucres que les enzymes dont ils dérivent. La substitution, la délétion ou l'addition d'un petit nombre d'acides aminés peut cependant aussi conduire à changer la spécificité enzymatique.

La sélection d'une séquence homologue est considérée comme évidente pour l'homme du métier, puisque l'on peut facilement créer des variants d'enzymes selon l'invention, en mettant en oeuvre des méthodes de mutagénèse classiques, et notamment en adaptant les méthodes décrites par Adams *et al.* (EP402450; Genencor), par Dunn *et al.* (Protein Engineering, 2, 283-291, 1988), par Greener *et al.* (Strategies, 7, 32-34, 1994) et/ou par Deng *et al.* (Anal. Biochem, 200, 81, 1992), par exemple.

Dans ce cadre, on considérera en particulier comme homologues deux séquences d'ADN qui, du fait de la dégénérescence du code génétique, codent pour un même polypeptide. De même, on peut aussi considérer comme homologue deux séquences d'ADN s'hybridant dans des conditions stringentes, c'est-à-dire s'hybridant toujours après une étape d'hybridation à 65°C pendant 15h dans un tampon d'hybridation (SSPE 1,5x: 0,225 M de NaCl, 0,0015 M d'EPTA, 0,015 M de NaH₂PO₄, pH7 ; 1% de SDS et 1% de lait déshydraté), suivie de 6 étapes successives de lavage à 65°C dans un tampon comprenant différentes dilutions de SSC 10x (1,5 M de NaCl, 0,15 M de citrate de sodium, pH7) et 0,1% SDS, respectivement pendant 2 fois 10 min avec du SSC 2x, pendant 2 fois 10 min avec du SSC 1x, et pendant 2 fois 5 min avec du SSC 0,1x.

On peut aussi considérer comme homologues les séquences d'ADN présentant plus de 50% d'identité avec les séquences selon l'invention, en particulier plus de 70%, l'identité étant déterminée par le rapport entre le nombre de bases nucléotidiques d'une séquence homologue qui sont identiques à celles d'une séquence selon l'invention, et le nombre total de bases nucléotidiques de ladite séquence selon l'invention, par exemple.

De même, on considérera comme homologues deux protéines fonctionnelles qui sont reconnues par un même anticorps, le rapport des valeurs d'intensité de reconnaissance des deux protéines par l'anticorps n'excédant pas 1000 dans un test ELISA, de préférence 100, par exemple. On considérera plus particulièrement comme homologues les séquences en acides aminés qui présentent plus de 70% d'identité avec les séquences selon l'invention, en particulier plus de 80% ou 90%. Dans ce dernier cas, l'identité est déterminée par le rapport entre le nombre d'acides aminés d'une séquence homologue qui sont identiques à celles d'une séquence selon l'invention, et le nombre total d'acides aminés de ladite séquence selon l'invention.

Pour isoler un ADN recombiné selon la présente invention, il est possible de constituer une banque de grands fragments d'ADN d'une bactérie lactique produisant un EPS dans une bactérie lactique ne produisant pas d'EPS, puis de sélectionner le ou les clone(s) produisant un EPS. Pour cela, on digère l'ADN génomique d'une bactérie lactique produisant un EPS par une enzyme de restriction qui est spécifique d'un site de restriction relativement rare (*Bam*HI*, Sal*I, *Pst*I) ou par une digestion partielle avec *Sau*3A, par exemple. On clone le produit de digestion dans un plasmide d'expression ou d'intégration qui accepte de grands fragments (plasmide pSA3, Dao *et al.*, Appl. Environ. Microbiol., 49, 115-119, 1985), on introduit les plasmides recombinants dans la même espèce de bactérie lactique ne produisant pas d'EPS, on sélectionne au moins un clone transformé produisant un EPS, puis on identifie, on isole et on séquence classiquement le fragment d'ADN responsable de la production d'EPS.

Vu que les ADN recombinés selon la présente invention sont susceptibles d'être de grande taille, du fait qu'ils peuvent contenir un groupe de gènes impliqués dans la biosynthèse d'EPS, on peut préférer introduire les plasmides recombinants dans la même souche de bactérie lactique dont provienne les fragments, à la différence près que cette souche a perdu la capacité de produire des EPS suite à un traitement mutagénique (traitement U.V., chimique ou par transposon).

Une alternative à la méthode décrite ci-dessus peut aussi consister à constituer une banque plasmidique de fragments d'ADN d'une souche de bactérie lactique produisant un EPS, à transformer la même souche de bactérie lactique par les plasmides incapables de s'y répliquer, à sélectionner les transformants ayant intégré un plasmide dans leur génome par recombinaison homologue (sélection par une résistance à un antibiotique, par exemple), à sélectionner les transformants ne produisant plus d'EPS, puis à isoler et séquencer les fragments d'ADN génomique des transformants sélectionnés qui sont adjacents au plasmide intégré. Pour cela, on peut digérer le chromosome des transformants, le liguer, puis effectuer une PCR-inverse à l'aide de sondes spécifiques du plasmide intégré ou introduire le produit de ligation dans une souche dans laquelle le plasmide recircularisé est capable de se répliquer, par exemple.

Une autre alternative à la méthode de sélection décrite ci-dessus peut aussi consister à transformer des bactéries lactiques produisant un EPS par un plasmide comprenant un transposon, à soumettre les bactéries à des conditions dans lesquelles le transposon s'excise du vecteur et s'intègre au hasard dans le génome, à sélectionner les clones de bactéries ayant perdu la capacité de produire des EPS, à isoler les fragments d'ADN génomique desdits clones dans lesquels un transposon s'est intégré. Cette méthode est décrite plus en détail par Stingele *et al.*, Dev. Biol. Stand., *In* Genetics of Streptococci, Enterococci and Lactococci, 85, 487-493, 1995 (ISSN:0301-5149).

Une autre alternative consiste à préparer des amorces nucléotidiques dérivées de gènes connus impliqués dans la biosynthèse d'un EPS, puis à réaliser une PCR sur l'ADN génomique d'une bactérie lactique produisant un EPS et pour laquelle on désire identifier les gènes impliqués dans la biosynthèse de cet EPS. Cette technique est cependant excessivement difficile à mettre en oeuvre, car le choix de la séquence nucléotidique des amorces sera déterminante dans l'isolement d'un gène. Puisque l'on ne connaît pas d'avance les homologies entre les gènes recherchés et les gènes connus, ce choix procède d'une démarche inventive, de par la nécessaire sélection opérée parmi une multiplicité d'amorces possibles.

Quelques critères peuvent cependant être retenus, tel que (1) la sélection d'une fonction enzymatique retrouvée souvent lors de la biosynthèse d'un EPS ; (2) la sélection de séquences nucléotidiques conservées en comparant des gènes de diverses bactéries codant pour cette fonction enzymatique; (3) la sélection de séquences conservées dont le contenu en nucléotides GC se rapproche le plus possible de l'organisme cible, même si pour cela on doit prendre comme point de départ de la sélection des séquences nucléotidiques retrouvées dans des organismes éloignés phylogéniquement de l'organisme cible (*E. coli*) ; (4) l'identification d'une partie dans les séquences conservées qui est naturellement plus riche en nucléotides GC ; (5) l'identification d'une partie dans les séquences conservées qui est peu dégénérée, c'est-à-dire codant pour des acides aminés qui sont eux-mêmes codés naturellement par 1 ou 2 codons, tel que la méthionine, le tryptophane, la glutamine ou l'acide glutamique, par exemple ; etc.

Il faut remarquer que les méthodes de sélection décrites brièvement ci-dessus peuvent être appliquées à toutes les bactéries lactiques connues, notamment aux bactéries lactiques mésophiles comme par exemple *Streptococcus cremoris, Streptococcus lactis, Lactobacillus casei* subsp. *casei, Lactobacillus sake,* et les bactéries lactiques thermophiles comme par exemple *Streptococcus thermophilus* et *Lactobacillus helveticus.* A cet effet, l'homme du métier dispose de techniques de transformation pour chaque espèce de bactérie lactique.

De plus, les méthodes de sélection décrites ci-dessus permettent le plus souvent d'isoler seulement une partie d'un gène ou d'un groupe de gènes impliqués dans la biosynthèse d'un EPS. Néanmoins, l'homme du métier peut facilement identifier la partie restante du gène ou du groupe de gènes en sélectionnant dans une banque génomique, à l'aide de sondes nucléotidiques basées sur un fragment isolé, un ou plusieurs clones renfermant la partie restante, par exemple.

On a pu ainsi caractériser une séquence d'ADN de 9,4 kb de la souche *Lactobacillus helveticus* LH59 qui a été déposée le 27 juillet 1994 selon le traité de Budapest auprès de la Collection Nationale de Culture de Microorganisme (C.N.C.M.), 28 rue du Docteur Roux, 75724 Paris cedex 15, France, où elle a reçu le numéro de dépôt CNCM I-1449. Par ailleurs, cette souche Gram-positif présente au microscope un aspect de bâtonnets non flagellées. Cette souche ne fait pas de spores, et elle est anaéorobe facultative. Toutes les restrictions concernant la disponibilité de cette souche seront irrévocablement levées lors de la délivrance d'un brevet correspondant à la présente demande ou une autre demande qui revendique le bénéfice de priorité sur cette demande.

Cette séquence de 9,4 kb comprend des gènes codant pour de nouvelles enzymes impliquées dans la synthèse de l'EPS ayant la structure répétée suivante:

β-D-Gal*p*-(1→4)-β-D-Glc*p* 1
↓
3
→5)-β-D-Gal*f*-(1→3)-α-D-Gal*p*-(1→3)-α-D-Glc*p*-(1→3)-β-D-Glc*p*-(1→

Grâce aux connaissances acquises sur la biosynthèse des EPS décrits dans la littérature, notamment de l'EPS décrit dans EP750043 (résultats non-publiés), on a pu ainsi identifier les nouvelles fonctions enzymatiques impliquées dans la biosynthèse de l'EPS ci-dessus. Les nouvelles enzymes impliquées dans la synthèse de cet EPS peuvent ainsi catalyser spécifiquement l'une des nouvelles liaisons suivantes entre sucres:
- la liaison osidique α-1,3 entre le carbone 1 d'un D-Glc*p* activé et le carbone 3 d'un Glc*p* appartenant à l'extrémité non-réductrice d'une chaîne saccharidique en formation, notamment la chaîne suivante: α/β-D-Glc*p*-amorce ;
- la liaison osidique α-1,3 entre le carbone 1 d'un D-Gal*p* activé et le carbone 3 d'un Glc*p* présent à l'extrémité non-réductrice d'une chaîne saccharidique en formation, notamment la chaîne suivante: α-D-Glc*p*-(1→3)-β/α-D-Glc*p*-amorce ;
- la liaison osidique β-1,3 entre le carbone 1 d'un D-Gal*f* activé et le carbone 3 d'un Gal*p* présent à l'extrémité non-réductrice d'une chaîne saccharidique en formation, notamment la chaîne suivante: α-D-Gal*p*-(1→3)-α-D-Glc*p*-(1→3)-β/α-D-Glc*p*-amorce;
- la liaison osidique β-1,3 entre le carbone 1 d'un D-Glc*p* activé et le carbone 3 d'un Gal*f* présent à l'extrémité non-réductrice d'une chaîne saccharidique en formation, notamment la chaîne suivante: β-D-Gal*f*-(1→3)-a-D-Gal*p*-(1→3)-α-D-Glc*p*-(1→3)-β-D-Glc*p*-amorce;
- la liaison osidique β-1,4 entre le carbone 1 d'un D-Gal*p* activé et le carbone 4 du Glc*p* présent à l'extrémité non-réductrice d'une chaîne saccharidique en formation, notamment la chaîne suivante: β-D-Glc*p*-(1→3)-β-D-Gal*f*-(1→3)-α-D-Gal*p*-(1→3)-α-D-Glc*p*-(1→3)-β-D-Glc*p*-amorce ; et,
- la liaison osidique β-1,5 entre les unités saccharidiques répétitives de l'EPS ci-dessus.

Les nouvelles enzymes recombinées, identifiées à partir de la séquence d'ADN de 9,4 kb, ont l'une des séquences en acides aminés choisie dans le groupe de séquences SEQ ID NO:2, SEQ ID NO:3, SEQ ID NO:4, SEQ ID NO:5, SEQ ID NO:6, SEQ ID NO:7, SEQ ID NO:8, et les séquences qui leur sont homologues.

La présente invention vise également tout ADN recombiné codant pour une enzyme selon l'invention, c'est à dire les enzymes choisies dans le groupe de séquences SEQ ID NO:2, SEQ ID NO:3, SEQ ID NO:4, SEQ ID NO:5, SEQ ID NO:6, SEQ ID NO:7, SEQ ID NO:8. En particulier, cet ADN peut comprendre au moins un gène ayant l'une des séquences nucléotidiques choisies parmi les séquences délimitées dans la séquence 1053-1730, 1734-2849, 2852-3943, 3930-5084, 5077-6096, 6099-7091, 7096-8259, 8284-8910 et les séquences qui leur sont homologues.

La présente invention vise aussi tout vecteur recombinant comprenant un ADN selon l'invention. Ce vecteur recombinant peut être tout fragment d'ADN, simple ou double brin, linéaire ou circulaire, d'expression ou d'intégration, et comprenant une séquence d'ADN selon l'invention, notamment tout ou partie de la séquence SEQ ID NO:1. Dans le cas où le procédé décrit dans EP564966 ne serait pas utilisé (voir ci-après), il faut veiller à ce que le vecteur exprime l'ADN selon l'invention par des séquences nucléotidiques adaptées (promoteur; site d'attachement du ribosome; codon préféré par l'organisme hôte, etc.), et le cas échéant à ce qu'il comprenne une ou plusieurs origines de réplication cellulaire, notamment d'*Escherichia coli* et/ou d'un *Lactobacillus helveticus,* par exemple.

Ainsi, pour opérer la biosynthèse d'un EPS, on peut intégrer tout ou partie de la séquence SEQ ID NO:1 comprenant au moins un des gènes précités dans une cellule hôte au moyen du procédé décrit dans EP564966, ledit procédé étant incorporé par référence dans l'enseignement de la présente invention. En résumé, ce procédé permet de pouvoir (1) transformer la cellule hôte avec un plasmide donneur qui ne s'y réplique pas, ledit plasmide comprenant ledit fragment placé fonctionnellement (le cadre de lecture est conservé) dans une partie d'un opéron issu de la cellule hôte; (2) identifier les transformants ayant intégré la totalité du plasmide; (3) sélectionner des transformants ayant uniquement intégré dans le chromosome le fragment selon l'invention, les autres séquences du plasmide s'étant excisées du chromosome; (4) et cultiver les transformants sélectionnés dans des conditions appropriées pour la production d'un EPS.

On peut noter que ce procédé permet de ne pas utiliser des séquences promotrice et d'activation traductionnelle fonctionnelles. De plus, les conditions de culture appropriées pour la production d'EPS sont à la portée de l'homme du métier, qui peut utiliser des milieux de culture standards, et choisir le pH, la température et l'agitation du milieu optimum selon la souche utilisée.

On peut aussi choisir de cloner tout ou partie de la séquence SEQ ID NO:1 comprenant au moins un des gènes précités dans un plasmide d'expression auto-réplicatif en aval de séquences promotrice et d'activation traductionnelle fonctionnelles, et le cas échéant en amont d'un terminateur, puis de transformer une cellule hôte par le plasmide recombinant.

On peut aussi utiliser les enzymes recombinées pour modifier ou synthétiser *in-vitro* un oligosaccharide ou un polysaccharide comme un EPS, par exemple. Pour cela, il est préférable de purifier au moins une de ces enzymes, en sur-exprimant classiquement leur gène dans une cellule, et en les isolant classiquement, par précipitation et/ou chromatographie (du milieu de culture et/ou de la partie membranaire des cellules), par exemple.

Un autre objet de la présente invention concerne une cellule comprenant, intégré dans son génome ou par le moyen d'un plasmide réplicable, un ADN recombiné selon l'invention, et exprimant une enzyme recombinée fonctionnelle selon l'invention. Cette cellule peut faire partie du groupe des moisissures, des levures, des bactéries et des plantes, par exemple. De préférence, les levures appartiennent aux genres *Saccharomyces, Kluyveromyces, Hansenula et Pichia ;* les bactéries sont Gram négative ou positive appartenant au genre *Escherichia, Bacillus, Lactobacillus, Lactococcus, Streptococcus et Staphylococcus* ; les cellules de plantes appartiennent aux légumineuses, et aux espèces céréalières et ligneuses ; tandis que les moisissures sont les cellules traditionnellement utilisées pour préparer un koji comme les *Aspergillus, Rhizopus et/ou Mucor.*

Un autre objet de la présente invention concerne un procédé de production d'un EPS, dans lequel (1) on clone dans un vecteur un fragment d'ADN codant pour au moins l'une des enzymes selon l'invention, ledit vecteur comprenant en outre une séquence permettant la réplication autonome ou l'intégration dans une cellule hôte ; (2) on transforme une cellule hôte par ledit vecteur, ladite cellule hôte utilisant les enzymes codées par le vecteur, le cas échéant en combinaison avec d'autres enzymes produites par la cellule hôte si le vecteur n'apporte pas tous les enzymes nécessaires à la biosynthèse d'un EPS, pour la biosynthèse d'un EPS ; (3) puis on cultive la cellule hôte transformée dans des conditions appropriées pour la production d'un EPS.

Si l'on ne met pas en oeuvre le procédé décrit dans EP564966, le vecteur doit alors comprendre en outre au moins une séquence promotrice fonctionnelle et au moins une séquence d'activation traductionnelle fonctionnelle, permettant l'expression des gènes codant pour au moins l'une des enzymes selon l'invention.

La présente invention a aussi pour objet un nouveau procédé de synthèse d'un EPS, qui est constitué par l'assemblage répété d'une seule unité saccharidique, elle-même formée d'une chaîne principale de sucres dont les extrémités sont impliquées dans la polymérisation des unités, dans lequel on utilise plusieurs enzymes recombinées ou plusieurs gènes recombinants susceptibles d'être impliqués dans la biosynthèse de l'EPS ayant l'unité saccharidique répétitive 
β-D-Galp-(1→4)-β-D-Glcp 1
↓
3
→5)-β-D-Galf-(1→3)-α-D-Galp-(1→3)-α-D-Glcp-(1→3)-β-D-Glcp-(1→
 caractérisé en ce que ces enzymes ou ces gènes, notamment choisis parmi les enzymes ou les gènes selon l'invention, permettent la création sur la chaîne principale de l'unité saccharidique d'une ou plusieurs ramifications lactose (β-D-Gal*p*-(1→4)-β-D-Glc*p*) liées à un sucre de la chaîne principale par leur glucose.

De préférence, la chaîne principale de l'unité saccharidique est constitué d'au moins 2 sucres choisis parmi D-Gal*p*, D-Gal*f* et/ou D-Glc*p*. De plus, le carbone 1 du glucose de la ramifications lactose peut être lié par une liaison osidique β à l'un des carbones 2-6 d'un des sucres de la chaîne principale, par exemple, de préférence le carbone 3 ou 6 d'un D-Gal*f.*

Lors de la mise oeuvre de ce procédé, on peut utiliser en outre une enzyme ou un gène qui permet la liaison entre un N-acétyl-galactosamine et le galactose de la ramification lactose. On peut aussi utiliser en outre une enzyme ou un gène qui permet la liaison entre l'acide N-acétyl-neuraminique et le galactose de la ramification lactose, par exemple.

Ce procédé de synthèse peut être mise en oeuvre *in vitro*, en utilisant les enzymes recombinantes et leurs substrats appropriés. On peut aussi le mettre en oeuvre *in vivo,* en clonant les gènes appropriés et en les faisant s'exprimer de sorte que les enzymes recombinées ainsi produites biosynthétisent un EPS en présence de leurs substrats, par exemple.

La présente invention a également pour objet un nouveau polysaccharide branché, constitué par l'assemblage répété d'une seule unité saccharidique, elle-même formée d'une chaîne principale de sucres dont les extrémités sont impliquées dans la polymérisation des unités, caractérisé en ce que la chaîne principale comprend au moins une ramification lactose liée à un sucre de la chaîne principale par son glucose, le galactose de cette ramification étant en outre lié à un N-acétyl-galactosamine.

Le carbone 1 du glucose de la ramifications lactose peut être lié par une liaison osidique β à l'un des carbones 2-6 d'un des sucres de la chaîne principale, de préférence le carbone 3 ou 6 d'un D-Gal*f* par exemple.

Pour des raisons de nouveauté, ce polysaccharide ne peut pas être constitué par la répétition de l'unité saccharidique suivante:

β-D-GalpNac-(1→4)-β-D-Galp-(1→4)-β-D-Glcp
1
↗
3
→5)-β-D-Galf-(1→3)-α-D-Galp-(1→3)-α-D-Glcp-(1→3)-β-D-Glcp(1→

En effet, le brevet NZ272795 (Société des Produits Nestlé) préconise déjà d'ajouter par voie enzymatique un N-acétylgalactosamine sur le galactose des ramifications contenues dans l'EPS de la souche LH59.

Pour obtenir ce nouveau polysaccharide, il suffit par exemple de mettre en oeuvre le procédé de synthèse décrit ci-dessus de sorte à obtenir un EPS différent de celui produit par la souche LH59, puis d'utiliser une β-1,4-N-acétylgalactosaminyltransférase en présence de son substrat, notamment celle décrite par Lutz *et al.* et Yamashiro *et al*. (J. of Biol. Chem., 269, 29227-29231, 1994; J. of Biol. Chem., 270, 6149-6155, 1995). On peut aussi purifier d'un milieu de culture un EPS différent de celui produit par la souche LH59, puis de lui adjoindre un N-acétylgalactosamine sur le galactose des ramifications lactose. L'EPS produit par la souche *L. helveticus* TY1-2 peut ainsi représenter une cible de choix (Y. Yamamoto *et al.,* Carbohydrate Research, 261, 67-78, 1994), par exemple. Cet EPS est constitué par la répétition de l'unité saccharidique suivante:

β-D-Galp-(1→4)-β-D-Glcp
1
↓
6
→6)-β-D-Glcp-(1→3)-β-D-Glcp-(1→6)-α-D-GlcpNac-(1→3)-β-D-Galp(1→
1
↘
4
β-D-Galp

[?]

Le nouveau polysaccharide selon l'invention peut également comprendre un acide N-acétyl-neuraminique lié sur le galactose de la ramification lactose. Pour cela, on peut utiliser une α-2,6-sialyltransférase, notamment celles décrites par Simth *et al*., Sjoberg *et al.* et/ou Yamamoto *et al*., (J. of Biol. Chem., 269, 15162-15171, 1994; J. Biochem., 271, 7450-7459, 1996; Biosci. Biotech. Biochem., 62, 210-214, 1998), ou une α-2,3-sialyltransférase, notamment celle commercialisée par Calbiochem® (Cat No.566218-M, USA), par exemple.

La présente invention est décrite plus en détail ci-après à l'aide du complément de description qui va suivre, qui se réfère à des exemples d'obtention de fragments d'ADN, de plasmides recombinants et de bactéries transformées selon l'invention. Ces exemples sont précédés d'une description des milieux de culture. Il va de soi, toutefois, que ces exemples sont donnés à titre d'illustration de l'objet de l'invention dont ils ne constituent en aucune manière une limitation. La manipulation de l'ADN, le clonage et la transformation de cellules bactériennes sont, en l'absence de précisions contraires, effectués selon les protocoles décrits dans l'ouvrage de Sambrook *et al.* (Molecular Cloning, A Laboratory Manual, Cold Spring Harbor Laboratory Press, U.S.A., 1989). Les pourcentages sont donnés en poids/poids, sauf indication contraire.

### Milieux: (rajouter 1,5% de Bacto-agar pour un milieu solide)

- M17 (Difco,USA): 0,5% de tryptone, 0,5% de soytone, 0,5% de viande hydrolysée, 0,25% d'extrait de levure, 0,05%, d'acide ascorbique, 0,025% de sulphate de magnésium, 1,9% de disodium-beta-glycérophosphate et de l'eau.
- LM17: milieu M17 comprenant 1% de lactose.
- GM17: milieu M17 comprenant 1% de glucose.
- MSK: lait écrémé (poudre reconstituée à 10%) comprenant 0,1% d'extrait de levure.
- MRS: protéose peptone 1%, extrait de boeuf 1%, extrait de levure 0,5%, dextrose 2%, tween 80 0,1%, citrate d'ammonium 0,2%, acétate d'ammonium 0,5%, phosphate de di-potassium 0,2%, sulfate de magnésium 0,1g/l, et sulfate de manganèse 0,05 g/l.

### Exemple I Clonage de l'operon de Lactobacillus helveticus CNCM I-1449 impliqué dans la synthèse d'un EPS

I.1. Structure répétée de l'EPS: la structure de l'EPS produit par la souche *Lactobacillus helveticus* LH59 (CNCM I-1449) a déjà été présentée par Stingele *et al.* (Carbohydrate Research, 302, 197-202, 1997), et dans la demande de brevet EP699689, le contenu technique de ces documents étant incorporé par référence dans la description de la présente invention.
I.222. Clonage: l'approche tentée afin d'isoler les gènes *eps* de la souche LH59 est basée sur la comparaison de gènes *eps* connus (ceux de *S. thermophilus*) et de leurs homologues (gènes *cap, cps, rfb*) d'espèces variées, et même d'espèces non-apparentées phylogéniquement, comme des espèces Gram-négatif. Ces comparaisons permettent ainsi de définir des régions conservées entre les espèces, et d'utiliser des amorces nucléotidiques dérivées de ces régions conservées afin d'amplifier par PCR une partie interne de l'opéron *eps* de la souche LH59. Cette approche s'est malheureusement heurtée à une impossibilité d'amplifier un gène *eps*, quel que soit les amorces choisies. Les raisons ayant conduit à ces échecs peuvent être multiples, par exemple liées à une faible homologie entre les gènes *eps* de la souche LH59 et ceux des régions conservées, ou à des conditions de PCR inadéquates, etc.
   Face à ces échecs, on a décidé de sélectionner, puis d'utiliser dans une PCR, des amorces nucléotidiques particulièrement dégénérées. Le choix de ces amorces spécifiques a en fait été déterminant pour l'amplification d'un gène *eps* de la souche LH59, et a été motivé par les considérations suivantes: (1) la sélection d'une fonction enzymatique retrouvée souvent lors de la biosynthèse d'un EPS ; (2) la sélection de séquences nucléotidiques conservées en comparant des gènes de diverses bactéries codant pour cette fonction enzymatique; (3) la sélection de séquences conservées dont le contenu en nucléotides GC se rapproche le plus possible de l'organisme cible, même si pour cela on doit prendre comme point de départ de la sélection des séquences nucléotidiques retrouvées dans des organismes éloignés phylogéniquement de l'organisme cible (*E. coli*) ; (4) l'identification d'une partie dans les séquences conservées qui est naturellement plus riche en nucléotides GC ; (5) l'identification d'une partie dans les séquences conservées qui est peu dégénérée, c'est-à-dire codant pour des acides aminés qui sont eux-mêmes codées naturellement par 1 ou 2 codons, tel que la méthionine, le tryptophane, la glutamine ou l'acide glutamique, par exemple ; etc.
   Parmi les amorces testés, celles ayant les séquences nucléotidiques SEQ ID NO:10 et SEQ ID NO:11, ont permis d'amplifier un fragment de gène *eps* dans les conditions de PCR suivantes: successivement 2 min à 95°C, 1 min à 42°C, 20 s à 72°C, puis 35 fois de suite le cycle de 30 s à 94°C, 1 min à 42°C et 20 s à 72°C. Pour cela, l'ADN génomique de la souche LH59 a été au préalable isolée selon la technique de Slos *et al.* (Appl. Environ. Microbiol., 57, 1333-1339, 1991). On utilise environ 100 ng d'ADN génomique et la Taq polymérase.
   Un fragment de PCR de 120 pb a ainsi pu être isolé, puis cloné dans le plasmide linéarisé pGEMT (Promega, USA). Le séquencage de ce fragment par la méthode des didéoxynucléotides (kit f-mol® DNA Sequencing System, Promega) indique une séquence correspondant aux nucléotides 1385 à 1526 de la séquence SEQ ID NO:1.
   Ce fragment de PCR a été utilisé pour cribler une banque λ-ZAP Express (Stratagene, USA) renfermant des fragments d'ADN de la souche LH59. Pour cela, selon les recommandations du fournisseur on digère partiellement une préparation d'ADN dudit mutant par *Sau*3A, on sépare les fragments par une électrophorèse sur gel d'agarose, on coupe du gel les bandes correspondantes à des fragments de 5 à 12 kb, on élue l'ADN, puis on le ligue au vecteur λ-ZAP Express préalablement digéré par *Bam*HI. On encapside *in-vitro* le produit de ligation à l'aide du système GigagoldIII (Stratagene), on mélange ensuite les phages avec des *Escherichia coli* XL1BIue (Stratagene) selon les recommandations du fournisseur, puis on étale le mélange sur boîte de Petri. On analyse ensuite les plaques recombinantes par hybridation de leur ADN transféré sur une membrane Hybond-N (Amersham Life Sciences, UK) avec le fragment de PCR préalablement rendus radioactifs (kit Random Primed DNA Labeling, Boehringer-Manheim).
   Parmi les nombreuses plaques recombinantes, on a pu sélectionner par hybridation plusieurs plaques positives, desquelles on a ensuite isolé les vecteurs λ-ZAP Express, puis excisé les vecteurs pBK-pCMV (pCMV) renfermant un insert génomique (voir les recommandations du fournisseur Stratagene). On a ensuite séquencé les inserts génomiques de ces vecteurs pCMV (kit f-mol® DNA Sequencing System). En recoupant les séquences nucléotidiques des différents inserts génomiques, on a pu ainsi caractériser une séquence de 9,4 kb correspondant à l'opéron de la souche LH59 impliqué dans la synthèse d'un EPS (voir la figure 1). La séquence nucléotidique de ce fragment de 9,4 kb est représentée dans la liste de séquence ci-après.
I.4. Analyse de la séquence SEQ ID NO:1: la séquence SEQ ID NO:1 contient l'opéron *eps* de la souche LH59. Cette séquence comprend 8 ORFs complets, dans la même orientation, que l'on appelle *eps1, eps2, eps3, eps4, eps5, eps6, eps7* et *eps8*, (voir figure 1). La comparaison des séquences en acides aminés codées par les ORFs avec celles de protéines présentes dans la banque de donnée Swiss-Prot, à l'aide des logiciels FASTA, PEPPLOT et PILEUP de GCG-softwear, Wisconsin, USA, permet de déduire la fonction de ces protéines. Les résultats sont présentés ci-après.

L'ORF *eps1* (nucléotides 1053-1730) code pour une protéine (SEQ ID NO:2) ayant environ 59% d'identité avec glycosyl-1-phosphate-transferase de *S. salivarius* (Genbank, n°P72577). Cette homologie confirme son rôle dans la biosynthèse de l'EPS.

L'ORF *eps2* (nucléotides 1734-2849) code pour une protéine (SEQ ID NO:3) ayant environ 36% d'identité avec une α-glycosyltransférase de *S. thermophilus* (Genbank, n°Q56044). Cette homologie confirme son rôle dans la biosynthèse de l'EPS.

L'ORF *eps3* (nucléotides 2852-3943) code pour une protéine (SEQ ID NO:4) ayant environ 32% d'identité avec une protéine de *Bacillus subtilis* (Genbank, n°P71055). Cet ORF code ainsi probablement pour une α-glycosyltransférase.

L'ORF *eps4* (nucléotides 3930-5084) code pour une protéine (SEQ ID NO:5) ayant environ 28% d'identité avec avec une protéine de *Bacillus subtilis* (Genbank, n°P71055). Cet ORF code probablement pour une α-glycosyltransférase.

L'ORF *eps5* (nucléotides 5077-6096) code pour une protéine (SEQ ID NO:6) ayant environ 29% d'identité avec une β-glycosyltransférase de *S. thermophilus* (GenBank n°O07339). Cette homologie confirme son rôle dans la biosynthèse de l'EPS.

L'ORF *eps6* (nucléotides 6099-7091) code pour une protéine (SEQ ID NO:7) ayant environ 29% d'identité avec une β-glycosyltransférase de *S. thermophilus* (GenBank n⁰O07339). Cette homologie confirme son rôle dans la biosynthèse de l'EPS.

L'ORF *eps7* (nucléotides 7096-8259) code pour une protéine (SEQ ID NO:8) ayant environ 27% d'identité avec une polymerase d'EPS de S. pneumaniae (GenBank n⁰O07867). Cette homologie confirme son rôle dans la biosynthèse de l'EPS.

L'ORF *eps8* (nucléotides 8284-8910) code pour une protéine (SEQ ID NO:9) ayant environ 27% d'identité avec des glycogenines (GenBank n°Q22997; P46976; P132780). Ces homologies confirment son rôle dans la biosynthèse de l'EPS.

En conclusion, les inserts génomiques isolés des vecteurs pCMV couvrent une région génomique de la souche *L. helveticus* LH59 qui est manifestement impliquée dans la biosynthèse de l'EPS.

### Exemple II; Inactivation des gènes eps

On inactive par recombinaison homologue les gène *eps1-8* de l'opéron pour confirmer leur importance dans la biosynthèse de l'EPS. Pour cela, on amplifie par PCR un fragment de chaque ORF provenant d'un des vecteurs pCMV décrits à l'exemple I ci-dessus, on ligue le produit de PCR dans le plasmide pSA3 préalablement digéré convenablement, on transforme la souche *E. coli* XL1-blue par le produit de ligation, on sélectionne des transformants, on isole un plasmide recombinant, puis on transforme la souche *L. helveticus* LH59 avec le plasmide recombinant au moyen de la méthode décrite par Thompson *et al.* (Appl. Micob. Biotec., 35, 334-338, 1991). On étale les cellules transformées sur un milieu solide MRS supplémenté de 2,5 µg/ml d'erythromycine, que l'on incube ensuite 16 h à 45°C, puis on sélectionne les colonies transformées qui survivent.

Les colonies qui survivent ont intégré dans un des 8 gènes *eps* le plasmide pSA3 recombinant. Ceci peut être vérifié par Southern-Blot d'une préparation d'ADN génomique des colonies survivantes digérée, et hybridation du filtre de Southern-Blot avec le produit de PCR rendu radioactif. Les colonies ayant intégré le plasmide présentent des bandes différentes que celles obtenues dans les mêmes conditions avec la souche sauvage. De plus, les colonies ayant intégré dans un des 8 gènes *eps* le plasmide recombinant présentent ont perdu leur caractère filant dans un lait MSK.

### Exemple III Biosynthèse d'un EPS

On prépare un plasmide à partir du plasmide pSA3 (Dao *et al*., Appl. Environ. Microbiol., 49, 115-119, 1985), en ajoutant un fragment d'ADN de la souche LH59 contenant la séquence de 9,4 kb décrite à l'exemple I (voir la liste de séquence), et en ajoutant les parties régulatrices de l'opéron *eps* de la souche *S. thermophilus* Sfi6 (EP750043), de sorte que l'expression (la transcription et la traduction) des gênes *eps* de la souche LH59 puisse être effectuée dans *S*. *thermophilus.* On transforme ensuite par électroporation, avec ce plasmide, la souche *S. thermophilus* CNCM I-1422 qui a été déposée le 18 mai 1994 selon le traité de Budapest. N'importe qu'elle autre souche filante *S. thermophilus* aurait pu aussi être utilisée. Cette souche présente au microscope un aspect de coques non flagellées formant des chaînettes, elle ne fait pas de spores, elle est anaéorobe facultative, et elle produit un EPS ayant la composition Glc:Gal=2:2.

### Exemple IV Biosynthèse d'un EPS

On prépare un plasmide à partir du plasmide pSA3 en ajoutant un fragment d'ADN de la souche LH59 contenant la séquence de 9,4 kb décrite à l'exemple I (voir la liste de séquence), et en ajoutant les parties régulatrices de l'opéron *eps* de la souche *S. thermophilus* Sfi6 (EP750043), de sorte que l'expression (la transcription et la traduction) des gènes *eps* de la souche LH59 puisse être effectuée dans *S. thermophilus*. On transforme ensuite par électroporation, avec ce plasmide, la souche *S. thermophilus* CNCM I-1351 qui a été déposée le 5 août 1993 selon le traité de Budapest. N'importe qu'elle autre souche filante *S. thermophilus* aurait pu aussi être utilisée. Cette souche présente au microscope un aspect de coques non flagellées formant des chaînettes, elle ne fait pas de spores, elle est anaéorobe facultative, et elle produit un EPS ayant la composition Glc:Gal:Rha=1:3:2

### Exemple V Biosynthèse d'un EPS

On isole de l'ADN génomique de la souche LH59 par le méthode de Slos *et al.* (Appl. Environ. Microbiol., 57, 1333-1339, 1991). On digère la préparation d'ADN par *Sal*I et *Bam*HI, on sépare les fragments d'ADN par électrophorèse sur gel d'agarose 0,7%, on élue les fragments supérieurs à 5 kb, on ligue l'ADN extrait au vecteur pJIM2279 (obtenu de P. Renault, INRA, Jouy-en-Josas, Paris, France) préalablement digéré par *Sal*I et *Bam*HI puis déphosphorylé. On transforme la souche *Lactococcus lactis* MG1363 (J. Bacteriol., 154, 1-9, 1983), cultivée sur milieu GM17 à 30°C, par la méthode de De Vos *et al*. (Gene, 85, 169-176, 1989). On sélectionne les clones transformés par hybridation du DNA génomique des clones avec l'une des sondes dérivées de la séquence SEQ ID NO:1. Parmi les transformants, quelques clones positifs sont sélectionnés.

### Exemple VI Préparation de gènes fonctionnels homologues aux gènes eps

On prépare des dérivés fonctionnels des gènes *eps1-8* en mettant en oeuvre une méthode adaptée de celle décrite par Adams *et al.* (EP402450; Genencor). Pour cela, on isole chacun des gènes *eps* par PCR à partir de l'ADN génomique de la souche LH59. Les amorces utilisées sont choisies à partir de la séquence SEQ ID NO:1, de sorte à amplifier uniquement chaque gène. On clone chaque produit de PCR dans le vecteur d'expression pQE60 (Qiagen, US) fonctionnellement en aval d'un promoteur *E. coli* régulant la transcription et la traduction du gène *eps*. On soumet enfin chaque vecteur à une mutagénèse chimique *in-vitro* avec de l'hydroxylamine, comme décrit par Adams *et al..*

On transforme la souche *E. coli* DH5α (Stratagen, US) qui ne possêde pas naturellement d'activité glycosyltransférase, par les plasmides pQE60 traités avec l'agent mutagène, puis on sélectionne des transformants.

Pour identifier parmi les transformants des gènes *eps* codant pour des variants d'enzymes, on analyse la spécificité enzymatique des enzymes exprimées dans chaque clone d'*E*.*coli,* au moyen de la méthode décrite à l'exemple VII. L'analyse des gène *eps* portés sur les plasmide pQE60 des clones ainsi sélectionnés montre que ceux-ci ne différent du gène *eps* original que par la substitution, la délétion ou l'addition d'un petit nombre de bases nucléotidiques conduisant à des modifications de la séquence en acides aminés de la glycosyltransférase.

### Exemple VII Synthèse de polysaccharides avec les glycosyltransférases de LH59

On isole chacun des gènes *eps* codant pour une glycosyltransférase par PCR à partir de l'ADN génomique de la souche LH59. Les amorces utilisées sont choisies à partir de la séquence SEQ ID NO:1, de sorte à amplifier uniquement chaque gène. On clone chaque produit de PCR dans un vecteur d'expression pQE60 (Qiagen, US). Les vecteurs ainsi traités sont ensuite introduits dans la souche *E. coli* DH5α. On cultive les souches DH5α transformées dans du milieu LB contenant de l'ampicilline jusqu'à une DO₆₀₀ de 0,8, on ajoute au milieu de culture 1 mM d'IPTG, on incube les cellules pendant 2 h, on centrifuge les cellules à 6000 g pendant 10 min à 4°C, on les lave dans le tampon A (50 mM de Tris-acétate pH8, 1mM de DTT, 1 mM d'EDTA et 10% de glycérol), on les centrifuge à nouveau, on re-suspend les cellules dans le tampon A contenant en outre 1 mM de PMSF (fluorure de phénylméthane sulfonyle) et on soumet la suspension à 15000 psi. On élimine les cellules entières par centrifugation à 6000 g pendant 15 min à 4°C, on récupère les membranes cellulaires du surnageant par ultracentrifugation à 100000 g pendant 1 h, et re-suspend l'aliquot dans le tampon A. Ce protocole permet ainsi généralement d'obtenir environ 10 mg/ml de protéines.

Parrallèlement, on cultive la souche LH59 dans un milieu contenant du D-Glc*p*, D-Gal*p* et D-Gal*f*, on soumet les cellules de cette culture à 20000 psi, on extrait des membranes les chaînes saccharidiques en formation (liées à une amorce) en traitant les débris cellulaires avec du chloroforme/méthanol puis en évaporant l'extrait. A titre de témoin dans les essais qui vont suivre, on procède de la même manière avec la souche LH59 cultivée en présence de sucres radioactifs.

On détermine ensuite la spécificité des glycosyltransférases présentes dans les membranes par un procédé similaire à celui décrit par Kolkman *et al.* (Mol. Microbiol., 26, 197-208, 1997). Pour cela, on fait réagir pendant 2 h, dans un volume réactionnel de 150 µl, 100 µl (environ 1 mg) de membranes, 50 mM de Tris-acétate pH8, 10 mM de MgCl₂, 1mM d'EDTA, 1 µl (environ 25 nCi) d'UDP^{[14C]}-Glc*p*, d'UDP^{[14C]-}Gal*p* ou d'UDP^{[14C]}-Gal*f* selon la glycosyltransférase considérée, et le dépôt contenant les chaînes saccharidiques en formation non-marquées isolées de la souche LH59 (voir le paragraphe ci-dessus). Pour arrêter la réaction, on ajoute au volume réactionnel 2 ml de chloroforme:méthanol (2:1), on agite vigoureusement le mélange, et on le laisse reposer pendant 30 min. On élimine les sucres nucléotidiques en extrayant la phase organique 3 fois de suite dans 0,4 ml de PSUP (pour 1 litre: 15 ml de chloroforme, 250 ml de méthanol, 1,83 g de KCl et 235 ml d'eau). La phase inférieure contenant toutes les chaînes saccharidiques liées à une amorce est ensuite séchée sous vide, resuspendue dans 200 µl de 1-butanol et séparée dans deux tubes.

Dans la première série de tubes, on détache les oligosaccharides de leur amorce en effectuant une hydrolyse douce dans 50 mM de TFA (ac. trifluoroacétique) à 90°C pendant 20 min.

Dans la deuxième série de tubes, on soumet les oligosaccharides à une hydrolyse poussée dans une solution de 4M TFA pendant 1 h à 125°C

On sèche ensuite les hydrolysats et on les resuspend dans 5 ml de sucres transporteurs dans 40% d'isopropanol (5 mg/ml de chaque sucre suivant: Glc, Gal, GalN, maltose, maltotriose, maltotetraose). Les sucres hydrolysées de chaque série de tubes sont alors déposés sur une plaque HPTLC silicagel 60 (Merck) que l'on soumet à un éluant composé de chloroforme, d'acide acétique et d'eau (respectivement 6:7:1). On autoradiographie ensuite la plaque pendant 16 à 24 h avec un film Biomax MS (Kodak). On visualise les sucres transporteurs en diffusant sur la plaque une solution d'éthanol comprenant 5% d'H₂SO₄, et en chauffant la plaque à 100°C pendant 15 min. L'analyse des plaques permet ensuite l'identification précise des fonctions enzymatiques codées par les gènes *eps* de la souche LH59.

### Exemple VIII Synthèse d'un nouveau polysaccharide

On purifie l'EPS d'un milieu de culture de la souche *Lactobacillus helveticus* TY1-2 (Y. Yamamoto *et al*., Carbohydrate Research, 261, 67-78, 1994). Cet EPS est constitué par la répétition de l'unité saccharidique suivante:

β-D-Galp-(1→4)-β-D-Glcp
1
↓
6
→6)-β-D-Glcp-(1→3)-β-D-Glcp-(1→6)-α-D-GlcpNac-(1→3)-β-D-Galp(1→
1
↘
4
β-D-Galp

Pour cela, on ajoute un volume d'acide trichloroacétique (40%) au milieu de culture, on centrifuge (17000 g, 20 min), on récupère le surnageant, on ajoute un volume d'acétone, on centrifuge à nouveau (17000 g, 20 min), on récupère la partie précipitée que l'on dissout dans de l'eau distillée à pH 7, on la dialyse dans de l'eau (12 h), puis on élimine les insolubles par ultracentrifugation (110000 g, 1 h).

Ensuite, on greffe un N-acétylgalactosamine sur le galactose des ramifications lactose de l'EPS, en utilisant une β-1,4-N-acétylgalactosaminyl-transférase et son substrat approprié, par exemple celles décrites par Lutz *et al.* et Yamashiro *et al.* (J. of Biol. Chem., 269, 29227-29231, 1994; J. of Biol. Chem., 270, 6149-6155, 1995).

### Exemple IX Synthèse d'un nouveau polysaccharide

On soumet l'EPS de l'exemple VIII à l'α-2,3-sialyltransférase commercialisée par Calbiochem® (Cat No.566218-M, USA) en présence de son substrat approprié.

### Example X Synthèse d'un nouveau polysaccharide

On soumet l'EPS de l'exemple VIII à l'α-2,6-sialyltransférase décrite par Sjoberg *et al.*, ou à l'α-2,6-sialyltransférase décrite par Yamamoto *et al.*, en présence de leurs substrats appropriés (J. Biochem., 271, 7450-7459, 1996; Biosci. Biotech. Biochem., 62, 210-214, 1998).

## Revendications

1. Enzyme recombinée, susceptible d'être impliquée dans la biosynthèse de l'EPS ayant l'unité saccharidique répétitive 
β-D-Galp-(1→4)-β-D-Glcp 1
↓
3
→5)-β-D-Galf-(1→3)-α-D-Galp-(1→3)-α-D-Glcp-(1→3)-β-D-Glcp-(1→
 catalysant spécifiquement l'une des liaisons suivantes entre sucres:
- la liaison osidique α-1,3 entre le carbone 1 d'un D-Glc*p* activé et le carbone 3 d'un Glc*p* appartenant à l'extrémité non-réductrice d'une chaîne saccharidique en formation
- la liaison osidique α-1,3 entre le carbone 1 d'un D-Gal*p* activé et le carbone 3 d'un Glc*p* présent à l'extrémité non-réductrice d'une chaîne saccharidique en formation ;
- la liaison osidique β-1,3 entre le carbone 1 d'un D-Gal*f* activé et le carbone 3 d'un Gal*p* présent à l'extrémité non-réductrice d'une chaîne saccharidique en formation ;
- la liaison osidique β-1,3 entre le carbone 1 d'un D-Glc*p* activé et le carbone 3 d'un Gal*f* présent à l'extrémité non-réductrice d'une chaîne saccharidique en formation ;
- la liaison osidique β-1,4 entre le carbone 1 d'un D-Gal*p* activé et le carbone 4 du Glc*p* présent à l'extrémité non-réductrice d'une chaîne saccharidique en formation ; et,
- la liaison osidique β-1,5 entre les unités saccharidiques répétitives de l'EPS ci-dessus.

2. Enzyme recombinée, notamment selon la revendication 1, ayant l'une des séquences en acides aminés choisie dans le groupe formé par les séquences SEQ ID NO:2, SEQ ID NO:3, SEQ ID NO:4, SEQ ID NO:5, SEQ ID NO:6, SEQ ID NO:7, SEQ ID NO:8, et les séquences qui leur sont homologues.

3. ADN recombiné codant pour une enzyme recombinée selon la revendication 1 ou 2.

4. ADN selon la revendication 3, comprenant au moins un gène ayant une séquence nucléotidique choisie parmi les séquences délimitées dans la séquence SEQ ID NO:1 par les nucléotides 1053-1730, 1734-2849, 2852-3943, 3930-5084, 5077-6096, 6099-7091, 7096-8259, 8284-8910 et les séquences qui leur sont homologues.

5. Vecteur recombinant comprenant un ADN selon la revendication 3 ou 4.

6. Cellule comprenant, intégré dans son génome ou par le moyen d'un plasmide réplicable, un ADN recombiné selon la revendication 3 ou 4, et exprimant une enzyme recombinée fonctionnelle selon la revendication 1 ou 2.

7. Cellule selon la revendication 6, caractérisée en ce qu'il s'agit d'une bactérie lactique.

8. Procédé de production d'un EPS, dans lequel (1) on clone dans un vecteur un fragment d'ADN codant pour au moins l'une des enzymes selon la revendication 1 ou 2, ledit vecteur comprenant en outre une séquence permettant la réplication autonome ou l'intégration dans une cellule hôte, (2) on transforme une cellule hôte par ledit vecteur, ladite cellule hôte utilisant les enzymes codées par le vecteur, le cas échéant en combinaison avec d'autres enzymes produite par la cellule hôte, pour la biosynthèse d'un EPS, (3) puis on cultive la cellule hôte transformée dans des conditions appropriées pour la production d'un EPS.

9. Procédé selon la revendication 8, dans lequel le vecteur comprend en outre au moins une séquence promoteur fonctionnelle et au moins une séquence d'activation traductionnelle fonctionnelle, permettant l'expression des ADN codant pour au moins l'une des enzymes selon la revendication 1 ou 2.

10. Procédé de synthèse d'un EPS, qui est constitué par l'assemblage répété d'une seule unité saccharidique, elle-même formée d'une chaîne principale de sucres dont les extrémités sont impliquées dans la polymérisation des unités, dans lequel on utilise plusieurs enzymes recombinées ou plusieurs gènes recombinants susceptibles d'être impliqués dans la biosynthèse de l'EPS ayant l'unité saccharidique répétitive 
β-D-Galp-(1→4)-β-D-Glcp 1
↓
3
→5)-β-D-Galf-(1→3)-α-D-Galp-(1→3)-α-D-Glcp-(1→3)-β-D-Glcp-(1→
 caractérisé en ce que ces enzymes ou ces gènes, notamment choisis parmi les enzymes revendiquées à la revendication 1 ou 2, ou les gènes revendiqués à la revendication 3 ou 4, permettent la création sur la chaîne principale de l'unité saccharidique d'une ou plusieurs ramifications lactose liées à un sucre de la chaîne principale par leur glucose.

11. Procédé selon la revendication 10, caractérisé en ce que la chaîne principale de l'unité saccharidique est constitué d'au moins 2 sucres choisis parmi D-Gal*p,* D-Gal*f* et/ou D-Glc*p.*

12. Procédé selon la revendication 10, caractérisé en ce que le glucose de la ramifications lactose est lié par une liaison osidique β-1,3 à un des sucres de la chaîne principale.

13. Procédé selon l'une des revendications 10 à 12, caractérisé en ce que l'on utilise en outre une enzyme ou un gène qui permet la liaison entre un N-acétyl-galactosamine et le galactose de la ramification lactose.

14. Procédé selon l'une des revendications 10 à 13, caractérisée en ce que l'on utilise en outre une enzyme ou un gène qui permet la liaison entre l'acide N-acétyl-neuraminique et le galactose de la ramification lactose.

15. Procédé selon l'une des revendications 10 ou 14, caractérisée en ce que la synthèse est effectuée *in vitro* et/ou *in vivo.*

16. Polysaccharide, susceptible d'être synthétisé selon le procédé revendiqué aux revendications 11 à 15, constitué par l'assemblage répété d'une seule unité saccharidique, elle-même formée d'une chaîne principale de sucres dont les extrémités sont impliquées dans la polymérisation des unités, caractérisé en ce que la chaîne principale comprend au moins une ramification lactose liée à un sucre de la chaîne principale par son glucose, le galactose de cette ramification étant en outre lié à un N-acétyl-galactosamine,
pour autant que ce polysaccharide ne consiste pas en la répétition de l'unité saccharidique suivante

β-D-GalpNac-(1→4)-β-D-Galp-(1→4)-β-D-Glcp
1
↗
3
→5)-β-D-Galf-(1→3)-α-D-Galp-(1→3)-α-D-Glcp-(1→3)-β-D-Glcp(1→

17. Polysaccharide branché selon la revendication 16, caractérisé en ce que le galactose de la ramification lactose est également lié à un acide N-acétyl-neuraminique.
